# EUROPEAN PATENT SPECIFICATION

(11) **EP 1 649 028 B1**
(45) Date of publication and mention of the grant of the patent: **22.08.2012**
(21) Application number: 04779179.3
(22) Date of filing: 26.07.2004
(51) Int. Cl.: C12N 15/861, A61K 39/235

(54) **ADENOVIRAL VECTOR-BASED VACCINES**
ADENOVIRALEVEKTOREN-BASIERTE IMPFSTOFFE
VACCINS A BASE DE VECTEURS ADENOVIRAUX

(30) Priority: 25.07.2003 US 490106 P; 14.07.2004 US 588000 P
(43) Date of publication of application: 26.04.2006
(73) Proprietor: GENVEC, INC., Gaithersburg, MD 20878 (US); The Government of the United States of America as represented by the Secretary of the Department of Health and Human Services, Rockville, MD 20852-3804 (US)
(72) Inventor: GALL, Jason G., D., Germantown, MD 20874 (US); WICKHAM, Thomas, J., Billerica, MA 01821 (US); ENRIGHT, William, J., North Potomac, MD 20878 (US); BROUGH, Douglas, E., Gaithersburg, MD 20879 (US); ZUBER, Mohammed, Frederick, MD 21703 (US); KING, C., Richter, Washington, DC 20010 (US); NABEL, Gary J., Bethesda, MD 20892 (US); CHENG, Cheng, Bethesda, MD 20892 (US)
(74) Representative: Wössner, Gottfried
(86) International application number: PCT/US2004/024002
(87) International publication number: WO 2005/012537

(56) References cited:
- WO-A-01/58940
- VOGELS R ET AL: "REPLICATION-DEFICIENT HUMAN ADENOVIRUS TYPE 35 VECTORS FOR GENE TRANSFER AND VACCINATION: EFFICIENT HUMAN CELL INFECTION AND BYPASS OF PREEXISTING ADENOVIRUS IMMUNITY" JOURNAL OF VIROLOGY, THE AMERICAN SOCIETY FOR MICROBIOLOGY, US, vol. 77, no. 15, August 2003 (2003-08), pages 8263-8271, XP009021328 ISSN: 0022-538X
- MEI Y-F ET AL: "Human Adenoviruses of Subgenera B, C, and E with Various Tropisms Differ in Both Binding to and Replication in the Epithelial A549 and 293 Cells" VIROLOGY, ACADEMIC PRESS,ORLANDO, US, vol. 295, no. 1, 30 March 2002 (2002-03-30), pages 30-43, XP004467141 ISSN: 0042-6822
- OSTAPCHUK PHILOMENA ET AL: "Pseudopackaging of adenovirus type 5 genomes into capsids containing the hexon proteins of adenovirus serotypes B, D, or E" JOURNAL OF VIROLOGY, vol. 75, no. 1, January 2001 (2001-01), pages 45-51, XP002320648 ISSN: 0022-538X
- MIYAZAWA N ET AL: "Fiber swap between adenovirus subgroups B and C alters intracellular trafficking of adenovirus gene transfer vectors." JOURNAL OF VIROLOGY. JUL 1999, vol. 73, no. 7, July 1999 (1999-07), pages 6056-6065, XP000957713 ISSN: 0022-538X
- GAO W ET AL: "Human adenovirus type 35: Nucleotide sequence and vector development." GENE THERAPY, vol. 10, no. 23, November 2003 (2003-11), pages 1941-1949, XP002320649 ISSN: 0969-7128
- HAVENGA M J E ET AL: "Exploiting the natural diversity in adenovirus tropism for therapy and prevention of disease" JOURNAL OF VIROLOGY, THE AMERICAN SOCIETY FOR MICROBIOLOGY, US, vol. 76, no. 9, May 2002 (2002-05), pages 4612-4620, XP002247093 ISSN: 0022-538X
- NAN X ET AL: "Development of an Ad7 cosmid system and generation of an Ad7DELTAE1DELTAE3HIVMN env/rev recombinant virus.", GENE THERAPY, vol. 10, no. 4, February 2003 (2003-02), pages 326-336, ISSN: 0969-7128

## Description

### FIELD OF THE INVENTION

This invention pertains to the use of a recombinant adenoviral vector in the manufacturing of a pharmaceutical composition for inducing an immune response in a mammal.

### BACKGROUND OF THE INVENTION

Delivery of therapeutics to sites of disease in biologically-relevant amounts has been an obstacle to drug development for decades. One solution that has proven to be a successful alternative to traditional drug delivery approaches is delivery of exogenous nucleic acid sequences for production of therapeutic factors *in vivo.* Gene transfer vectors ideally enter a wide variety of cell types, have the capacity to accept large nucleic acid sequences, are safe, and can be produced in quantities required for treating patients. Adenoviral vectors have all of these advantageous properties and are used in a variety of protocols to treat or prevent biological disorders.

However, widespread use of adenoviral vectors is hindered, at least in part, by the immunogenicity of the vector. A majority of the U.S. population has been exposed to wild-type adenovirus and developed pre-existing immunity to adenovirus-based gene transfer vectors. As a result, adenoviral vectors are quickly cleared from the bloodstream, thereby reducing the effectiveness of the vector in delivering biologically-relevant amounts of gene product. The neutralization and/or clearance of adenoviral vectors in the body complicates use of these vectors as DNA vaccines. DNA vaccines employ gene transfer vectors to deliver antigen-encoding DNA to host cells. By producing antigenic proteins *in vivo*, the humoral and cell-mediated arms of the immune system are activated, thereby generating a more complete immune response against the antigen as compared to traditional vaccines wherein foreign proteins are injected into the body. Despite the advantageous characteristics of adenoviral vectors as gene delivery vehicles, the immunogenicity of the vector prevents efficient repeat dosing, which can be advantageous for "boosting" the immune system against pathogens, and results in only a small fraction of a dose of adenoviral vector delivering its payload to host cells.

The therapeutic use of adenoviral vectors also is limited by the stability of adenoviral vectors. For example, removal of the E1 region of the serotype 5 adenoviral genome to render the adenoviral replication-deficient often results in downregulation of the capsid protein IX (pIX) expression. While downregulation of pIX does not appear to inhibit production of viral particles, adenoviral vectors deficient in pIX have been shown to be thermolabile *in vivo* (see, e.g. Colby et al., J. Virol., 39, 977-980 (1981)), and cannot package full-length genomes (see, e.g., Ghosh-Choudhury et al., EMBO J., 6, 1733-1739 (1987), and Caravokyri et al., J. Virol., 69, 6627-6633 (1995)).

Accordingly, there is a need in the art for alternative adenoviral vector constructs and methods of using such constructs to deliver nucleic acid sequences, particularly antigen-encoding nucleic acid sequences, to host cells. The invention provides such an adenoviral vector and methods of use. These and other advantages of the invention, as well as additional inventive features, will be apparent from the description of the invention provided herein.

### BRIEF SUMMARY OF THE INVENTION

The invention pertains to the use of a non-subgroup C adenoviral vector according to claim 1.

The invention further provides the use of a composition comprising (a) a serotype 41 or a serotype 35 adenoviral vector comprising an adenoviral genome deficient in one or more replication-essential gene functions of the E1A region of the adenoviral genome and the E1B region of the adenoviral genome encoding the E1B 55K protein and (b) a carrier. In addition, the invention provides a method of producing an adenoviral vector. The method comprises introducing a serotype 41 adenoviral vector or a serotype 35 adenoviral vector comprising an adenoviral genome deficient in one or more replication-essential gene functions of the E1A region of the adenoviral genome and the E1B region of the adenoviral genome encoding the E1B 55K protein into a cell comprising a subgroup C adenoviral nucleic acid sequence encoding the one or more replication-essential gene functions of the E1A region and E1B region which are deficient in the adenoviral vector. The cell further comprises open reading frame 6 (ORF6) of a subgroup C adenoviral E4 region. The cell does not comprise a non-subgroup C nucleic acid sequence encoding the one or more gene functions of the E1A region and E1B region deficient in the adenoviral vector. The method further comprises propagating the adenoviral vector.

### BRIEF DESCRIPTION OF THE DRAWINGS

Figure 1 is a graph illustrating the virus titer of the adenoviral vectors Ad35f and Ad35P5 over time at 48° C.

Figure 2 is a graph illustrating the results of mass spectroscopic analysis of wild type Ad35 and an E1-deleted Ad35 adenoviral vector.

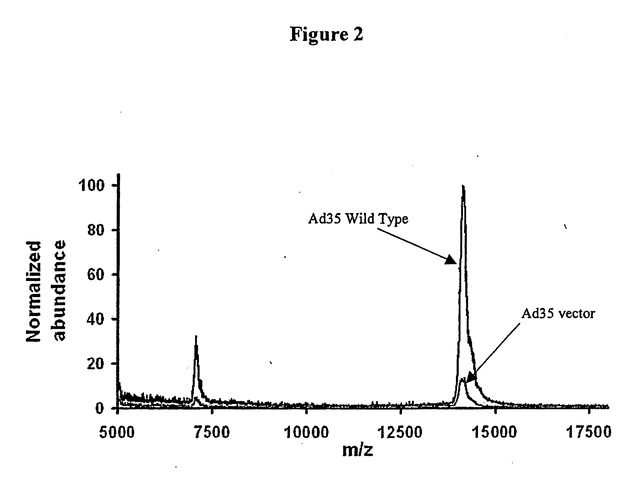

Figure 2 is a diagram illustrating the genome of an Ad35 adenoviral vector comprising the nucleic acid sequence encoding pIX operably linked to an AAV p5 promoter.

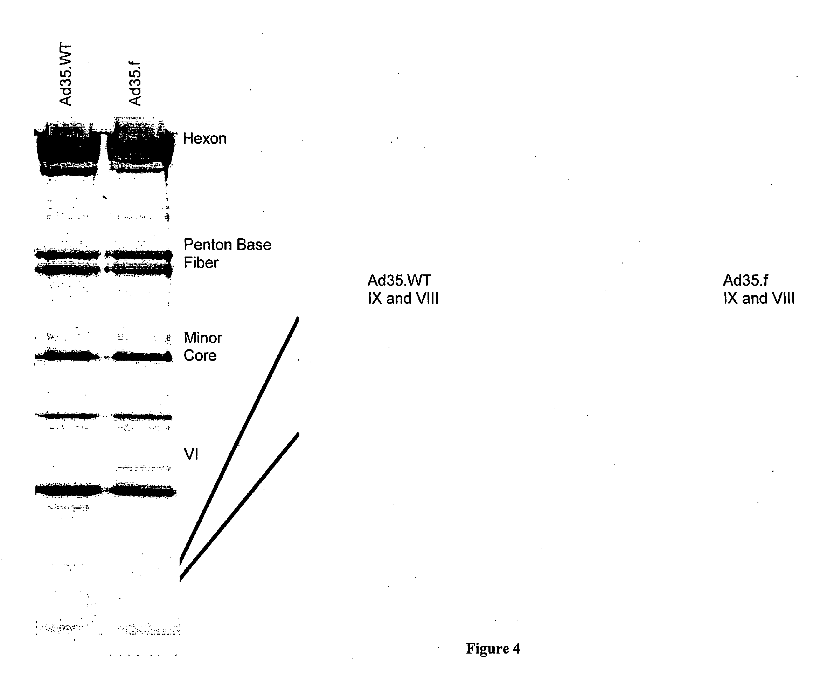

Figure 4 depicts a silver-stained polyacrylamide gel analysis of the proteins encoded by wild type Ad35 and the adenoviral vector Ad35f.

### DETAILED DESCRIPTION OF THE INVENTION

The invention provides materials and their use for inducing an immune response in a mammal. In particular, the invention provides adenoviral vectors suited for delivering nucleic acid sequences encoding one or more antigens to host cells and methods of using such adenoviral vectors to induce an immune response against one or more encoded antigens. In one embodiment, the invention provides the use of an adenoviral vector for inducing an immune response in a mammal, comprising administering to the mammal a non-subgroup C adenoviral vector comprising an adenoviral fiber protein comprising an amino acid sequence comprising about 80% or more identity to an amino acid sequence encoding a subgroup C adenoviral fiber protein. The adenoviral vector further comprises a nucleic acid sequence encoding at least one antigen which is expressed in the mammal to induce an immune response.
Adenovirus from any origin, any subgroup, mixture of subgroups, or any chimeric adenovirus can be used as the source of the viral genome for the adenoviral vector of the invention. A human adenovirus preferably is used as the source of the viral genome for an adenoviral vector delivered to human patients. In this regard, the adenovirus can be of subgroup C (e.g., serotypes 2 and 5). In the context of the inventive method, however, the adenovirus preferably is not a subgroup C adenovirus. For instance, an adenovirus can be of subgroup A (e.g., serotypes 12, 18, and 31), subgroup B (e.g., serotypes 3, 7, 11, 14, 16, 21, 34, 35, and 50), subgroup D (e.g., serotypes 8, 9, 10, 13, 15, 17, 19, 20, 22-30, 32, 33, 36-39, and 42-48), subgroup E (e.g., serotype 4), subgroup F (e.g., serotypes 40 and 41), or an unclassified subgroup (e.g., serotypes 49 and 51). Adenoviral serotypes 1 through 51 are available from the American Type Culture Collection (ATCC, Manassas, VA). Preferably, the adenoviral vector is a subgroup B or subgroup F adenoviral vector. More preferably, the adenoviral vector is a serotype 35 adenoviral vector or a serotype 41 adenoviral vector. Non-subgroup C adenoviral vectors, as well as methods of producing non-subgroup C adenoviral vectors, are disclosed in, for example, U.S. Patents 5,801,030; 5,837,511; and 5,849,561 and International Patent Applications WO 97/12986 and WO 98/53087.

An adenovirus used as the source of the viral genome for an adenoviral vector need not be a human adenovirus. Canine, ovine, avian, bovine, or simian (non-human) adenovirus can serve as adenoviral vectors. Adenoviral vectors based on virus isolated from non-human animals likely will evade pre-existing immunity as human patients are not naturally infected by these viruses. Non-human adenoviral vectors are further described in U.S. Patents 6,083,716 and 6,479,290 and U.S. Patent Application Publications 2003/009678 A1 and 2003/0108569 A1.

Adenovirus from different subgroups vary as to immunologic reactivity, oncogenicity, and transduction efficiencies for different cell types. Adenovirus, in general, can infect a wide variety of cell types. In nature, however, adenovirus of different subgroups can infect different tissues in the body, e.g., the gut versus respiratory tissues. The inventive method capitalizes on the differences of non-subgroup C adenoviruses compared to subgroup C adenoviruses commonly used for *in vivo* gene transfer and to which a majority of the population has pre-existing immunity. Non-subgroup C adenoviral vectors are not neutralized in mammals with pre-existing immunity to subgroup C adenoviral vectors as quickly as subgroup C adenoviral vectors, which allows greater circulation time and increased transduction efficiency (Vogels et al., Journal of Virology, 77(15), 8263-8271 (2003)). In addition, the natural tropicity of non-subgroup C adenoviral vectors can facilitate antigen delivery to a desired target tissue.

However, non-subgroup C adenoviral vectors can comprise unique attributes which render them undesirable or, at most, less desirable than subgroup C adenoviral vectors in the context of DNA vaccines. Therefore, while the serotype 35 adenoviral vector successfully evades preexisting immunity to adenovirus, the adenoviral vector could downregulate, block, or fail to stimulate an immune response against a desired antigen. The invention seeks to overcome the obstacles associated with using non-subgroup C adenoviral vectors as DNA vaccine vehicles.

Non-subgroup C adenoviral vectors unable to mediate an immune response against an encoded antigen typically differ from subgroup C adenoviral vectors in three respects: tropism for cell surface receptors, viral entry mechanism, and genome. Some adenovirus serotypes use different cell surface receptors to enter host cells. For example, adenoviral subgroup C serotype 5 and serotype 2 adenoviral vectors bind the coxsackievirus and adenovirus receptor (CAR), which, in combination with cell surface integrins, mediates viral entry into the host cell. Subgroup C adenovirus also can bind the major histocompatability complex-1 (MHC I) α2 domain and heparin sulfate glycosaminoglycans via the knob region and shaft region of the fiber protein, respectively (see, e.g., Hong et al., EMBO J.,16, 2294-2306 (1997), and Dechecchi et al., J. Virol., 75, 8772-8780 (2001)). In contrast, subgroup B2 adenoviral vectors, such as adenoviral serotype 35 vectors, naturally bind CD46, a cell surface complement protein, to gain entry into host cells (see, for example, Gaggar et al., Molecular Therapy, 7(5), Abstract 416 (2003)). Blocking binding of a non-subgroup C adenoviral vector to its native cell surface receptor (e.g., a complement protein, such as CD46) can block the vector-mediated negative regulation of an immune reaction. Likewise, modification of the viral entry mechanism including, for example, use of a non-native receptor to enter cells, ablation of native binding and/or retargeting of the adenoviral capsid to cell surface accessory molecules which facilitate virus-cell binding (e.g., αᵥ integrins), and/or redesignation of intracellular pathways involved in viral cycling to the nucleus, also can inhibit non-subgroup C adenoviral vector-mediated downregulation of a mammal's immune reaction to an encoded antigen. On the other hand, retargeting a non-subgroup C adenoviral vector capsid, which does not bind a cell-surface receptor for a subgroup C adenoviral vector (e.g., CAR, MHC I α2 domain, or heparin sulfate glycosaminoglycans, to enter host cells via subgroup C cell-surface receptor binding (e.g., by insertion of a CAR-binding non-native amino acid sequence into the fiber protein, use of a bi-specific molecule, and the like) can inhibit or prevent downregulation of the immune response mediated by the non-subgroup C adenoviral vector or upregulate (i.e., enhance or activate) a mammal's immune reaction to an encoded antigen.

To this end, the non-subgroup C adenoviral vector of the inventive method preferably comprises an adenoviral fiber protein comprising an amino acid sequence comprising about 80% or more identity to an amino acid sequence encoding an adenoviral fiber protein that binds CAR to mediate virus entry into a host cell. Adenoviral fiber proteins that bind CAR are described in, for example, International Patent Application WO 00/15823. Ideally, the adenoviral fiber protein of the non-subgroup C adenoviral vector comprises an amino acid sequence comprising about 80% or more identity to an amino acid sequence encoding a subgroup C adenoviral fiber protein. Even more preferably, the non-subgroup C adenoviral vector comprises subgroup C adenoviral fiber proteins (e.g., serotype 5 adenoviral fiber protein) incorporated into the viral capsid. The inclusion of an adenoviral fiber protein comprising about 80% or more identity to a subgroup C adenoviral fiber protein serves to ablate native binding of the non-subgroup C adenoviral vector, redirect the adenoviral vector to CAR, and serves as an adjuvant in enhancing the immune response to the encoded antigen. Subgroup C adenovirus includes adenoviral serotypes 1, 2, 5, and 6. A native subgroup C adenoviral fiber protein can be incorporated into the adenoviral surface unmodified or can be modified as desired by the practitioner. Alternatively, a synthetic fiber protein can be generated. In any event, the amino acid sequence of the adenoviral fiber protein comprises about 80% or more identity (e.g., about 85% or more identity, or about 90% or more identity) to an amino acid sequence encoding a CAR-binding adenoviral fiber protein, desirably a subgroup C adenoviral fiber protein. Preferably, the adenoviral fiber protein comprises an amino acid sequence comprising about 95% or more identity (e.g., 100% identity) to an amino acid sequence encoding a subgroup C adenoviral fiber protein.

"Identity" with respect to amino acid or polynucleotide sequences refers to the percentage of residues or bases that are identical in the two sequences when the sequences are optimally aligned. If, in the optimal alignment, a position in a first sequence is occupied by the same amino acid residue or nucleotide as the corresponding position in the second sequence, the sequences exhibit identity with respect to that position. The level of identity between two sequences (or "percent sequence identity") is measured as a ratio of the number of identical positions shared by the sequences with respect to the size of the sequences (i.e., percent sequence identity = (number of identical positions/total number of positions) x 100). A number of mathematical algorithms for rapidly obtaining the optimal alignment and calculating identity between two or more sequences are known and incorporated into a number of available software programs. Examples of such programs include the MATCH-BOX, MULTAIN, GCG, FASTA, and ROBUST programs for amino acid sequence analysis, and the SIM, GAP, NAP, LAP2, GAP2, and PIPMAKER programs for nucleotide sequences. Preferred software analysis programs for both amino acid and polynucleotide sequence analysis include the ALIGN, CLUSTAL-W (e.g., version 1.6 and later versions thereof), and BLAST programs (e.g., BLAST 2.1, BL2SEQ, and later versions thereof).

Alternatively, the nucleic acid sequence encoding the adenoviral fiber protein can be similar enough to a nucleic acid sequence encoding the subgroup C adenoviral fiber protein to hybridize under at least moderate, preferably high, stringency conditions, and retain biological activity. Exemplary moderate stringency conditions include overnight incubation at 37° C in a solution comprising 20% formamide, 5x SSC (150 mM NaCl, 15 mM trisodium citrate), 50 mM sodium phosphate (pH 7.6), 5x Denhardt's solution, 10% dextran sulfate, and 20 mg/ml denatured sheared salmon sperm DNA, followed by washing the filters in 1x SSC at about 37-50° C, or substantially similar conditions, e.g., the moderately stringent conditions described in Sambrook et al., Molecular Cloning, a Laboratory Manual, 2d edition, Cold Spring Harbor Press, Cold Spring Harbor, N.Y. (1989), and Ausubel et al., Current Protocols in Molecular Biology, Greene Publishing Associates and John Wiley & Sons, New York, N.Y. (1994). High stringency conditions are conditions that use, for example, (1) low ionic strength and high temperature for washing, such as 0.015 M sodium chloride/0.0015 M sodium citrate/0.1% sodium dodecyl sulfate (SDS) at 50° C, (2) employ a denaturing agent during hybridization, such as formamide, for example, 50% (v/v) formamide with 0.1% bovine serum albumin (BSA)/.01 % Ficoll/0.1 % polyvinylpyrrolidone (PVP)/50 mM sodium phosphate buffer at pH 6.5 with 750 mM sodium chloride, 75 mM sodium citrate at 42° C, or (3) employ 50% formamide, 5x SSC (0.75 M NaCl, 0.075 M sodium citrate), 50 mM sodium phosphate (pH 6.8), 0.1% sodium pyrophosphate, 5x Denhardt's solution, sonicated salmon sperm DNA (50 mg/ml), 0.1% SDS, and 10% dextran sulfate at 42° C, with washes at (i) 42° C in 0.2x SSC, (ii) at 55° C in 50% formamide, and (iii) at 55° C in 0.1x SSC (preferably in combination with EDTA).

On the other hand, the adenoviral vector need not comprise an entire fiber protein wherein the amino acid sequence is 80% or more identical to an amino acid sequence of an intact adenoviral fiber protein that binds CAR, e.g., a subgroup C adenoviral fiber protein. In a separate embodiment, the adenoviral vector can comprise a chimeric adenoviral fiber protein, at least a portion of which comprises an amino acid sequence that is native to the adenoviral vector genome or an amino acid sequence that is 80% or more identical to an amino acid sequence encoding an adenoviral fiber protein of the non-subgroup C adenovirus (e.g., an adenovirus of the same subgroup and, optionally, serotype) as the adenoviral vector genome. In this regard, the chimeric adenoviral fiber protein also comprises an amino acid sequence of a subgroup C adenoviral fiber protein. To illustrate, but not limit the scope of the invention, a serotype 35 (or serotype 41) adenoviral vector can be constructed comprising an adenoviral fiber having a tail, shaft, and knob region. The adenoviral fiber tail region is native to the adenoviral vector, while the shaft and knob regions are adenoviral serotype 5 fiber regions. Alternatively, the adenoviral fiber comprises adenoviral serotype 35 tail and shaft regions and an adenoviral serotype 5 fiber knob. The chimeric fiber protein can be further modified as described herein to, for example, increase antigenicity, modulate tropicity, and/or display antigenic epitopes on the viral surface.

Adenoviral serotype 41 comprises two distinct types of fiber protein, a long fiber protein and a short fiber protein, incorporated into the viral capsid. Accordingly, one or both of the fiber proteins of adenovirus serotype 41 vectors can be modified as described herein. For example, the long serotype 41 adenoviral fiber protein, which binds CAR, can be retained while the short serotype 41 adenoviral fiber is replaced with a serotype 5 or serotype 2 adenoviral fiber protein. Alternatively, the short fiber can be engineered to be a chimeric adenoviral serotype 41- adenoviral serotype 5 fiber as described above. Further, the short fiber can be engineered to include a ligand, such as an RGD-containing ligand, to target specific cell types. Serotype 41 adenoviral vectors provide unique options in the context of the inventive method with respect to increasing immunogenicity of the vector while retaining native tropism. This can be achieved not only by exchanging the short fiber protein with another fiber protein or creating a chimeric fiber protein, but also by modifying the fiber protein to create a chimeric adenoviral fiber-antigen protein, whereby the fiber-antigen chimeric protein provokes an immune response to the antigen.

In addition to the difference(s) in viral entry mechanisms, non-subgroup C and subgroup C adenoviral vectors differ as to their respective genomes. For example, the E3 region of serotype 35 adenovirus comprises at least two additional coding sequences than the E3 region of serotype 5 adenovirus (see, for example, Vogels et al., J. Virol., 77(15), 8263-8271 (2003)). The coding sequences unique to non-subgroup C adenovirus (which a subgroup 5 adenovirus does not share) also can be responsible for downregulating a mammal's immune reaction to an encoded antigen. The gene product(s) encoded by the early gene region E1A, the delayed early gene regions E1B, E2, E3, and E4, or the late regions L1-L5 of the adenoviral genome can induce the host cell to produce factors which mask the encoded antigen, alter the cellular environment to prevent or distort antigen presentation to immune effector cells, stimulate production of immuno-repressors, inhibit production of immunostimulants, and the like. To create non-subgroup C adenoviral vectors for inducing an immune response in a mammal against an encoded antigen, the adenoviral vector can be rendered deficient in one or more gene functions of the early or late regions of the adenoviral genome by, for example, excision or replacement of the relevant gene regions. Alternatively, one or more regions of the adenoviral vector genome (e.g., the E1A, E1B, E2A, E3, E4, L1, L2, L3, L4, and/or L5 regions) can be replaced by corresponding regions from a subgroup C adenoviral genome (preferably a serotype 5 adenoviral genome). A preferred adenoviral vector construct (e.g., for vaccine development) comprises a non-subgroup C adenoviral vector genome (e.g., a serotype 35 or serotype 41 adenoviral vector genome) wherein the E3 and/or E4 region of the adenoviral genome is replaced with the corresponding region(s) of a serotype 5 adenoviral genome. Yet, the ability of certain non-subgroup C adenoviral vectors to evade pre-existing immunity and downregulate an immune response against transgene products can be advantageous outside of vaccine development for delivering an exogenous nucleic acid sequence to, for example, the eye, inner ear, lymph nodes, brain, and heart, to treat or prevent biological disorders.

The adenoviral vector of the invention can be replication competent. For example, the adenoviral vector can have a mutation (e.g., a deletion, an insertion, or a substitution) in the adenoviral genome that does not inhibit viral replication in host cells. Preferably, however, the adenoviral vector is replication-deficient. By "replication-deficient" is meant that the adenoviral vector comprises an adenoviral genome that lacks at least one replication-essential gene function (i.e., such that the adenoviral vector does not replicate in typical host cells, especially those in a human patient that could be infected by the adenoviral vector in the course of the inventive method). A deficiency in a gene, gene function, or gene or genomic region, as used herein, is defined as a deletion of sufficient genetic material of the viral genome to impair or obliterate the function of the gene whose nucleic acid sequence was deleted in whole or in part. While deletion of genetic material is preferred, mutation of genetic material by addition or substitution also is appropriate for disrupting gene function. Replication-essential gene functions are those gene functions that are required for replication (e.g., propagation) and are encoded by, for example, the adenoviral early regions (e.g., the E1, E2, and E4 regions), late regions (e.g., the L1-L5 regions), genes involved in viral packaging (e.g., the IVa2 gene), and virus-associated RNAs (e.g., VA-RNA1 and/or VA-RNA-2). More preferably, the replication-deficient adenoviral vector comprises an adenoviral genome deficient in at least one replication-essential gene function of one or more regions of the adenoviral genome. Preferably, the adenoviral vector is deficient in at least one gene function of the E1A region, the E1B region, or the E4 region of the adenoviral genome required for viral replication (denoted an E1-deficient or E4-deficient adenoviral vector). In addition to a deficiency in the E1 region, the recombinant adenovirus also can have a mutation in the major late promoter (MLP), as discussed in International Patent Application WO 00/00628. Most preferably, the adenoviral vector is deficient in at least one replication-essential gene function (desirably all replication-essential gene functions) of the E1 region and at least one gene function of the nonessential E3 region (e.g., an Xba I deletion of the E3 region) (denoted an E1/E3-deficient adenoviral vector). With respect to the E1 region, the adenoviral vector can be deficient in all or part of the E1A region and all or part of the E1B region. To illustrate but not limit this embodiment, a serotype 35 adenoviral vector can comprise an E1 deletion of nucleotides 570 to 3484. When the adenoviral vector is deficient in at least one replication-essential gene function in only one region of the adenoviral genome (e.g., an E1- or E1/E3-deficient adenoviral vector), the adenoviral vector is referred to as "singly replication-deficient."

The adenoviral vector of the invention can be "multiply replication-deficient," meaning that the adenoviral vector is deficient in one or more replication-essential gene functions in each of two or more regions of the adenoviral genome. For example, the aforementioned E1-deficient or E1/E3-deficient adenoviral vector can be further deficient in at least one replication-essential gene function of the E4 region (denoted an E1/E4- or E1/E3/E4-deficient adenoviral vector), and/or the E2 region (denoted an E1/E2- or E1/E2/E3-deficient adenoviral vector), preferably the E2A region (denoted an E1/E2A- or E1/E2A/E3-deficient adenoviral vector).

By removing all or part of, for example, the E1, E3, and E4 regions of the adenoviral genome, the resulting adenoviral vector is able to accept inserts of exogenous nucleic acid sequences while retaining the ability to be packaged into adenoviral capsids. The nucleic acid sequence can be positioned in the E1 region, the E3 region, or the E4 region of the adenoviral genome. Indeed, the nucleic acid sequence can be inserted anywhere in the adenoviral genome so long as the position does not prevent expression of the nucleic acid sequence or interfere with packaging of the adenoviral vector. The adenoviral vector also can comprise multiple (i.e., two or more) nucleic acid sequences encoding the same antigen. Alternatively, the adenoviral vector can comprise multiple nucleic acid sequences encoding two or more different antigens. Each nucleic acid sequence can be operably linked to the same promoter, or to different promoters depending on the expression profile desired by the practitioner, and can be inserted in the same region of the adenoviral genome (e.g., the E4 region) or in different regions of the adenoviral genome.

The adenoviral vector, when multiply replication-deficient, especially in replication-essential gene functions of the E1 and E4 regions, preferably includes a spacer sequence to provide viral growth in a complementing cell line similar to that achieved by singly replication-deficient adenoviral vectors, particularly an E1-deficient adenoviral vector. The spacer sequence can contain any nucleotide sequence or sequences which are of a desired length, such as sequences at least about 15 base pairs (e.g., between about 15 base pairs and about 12,000 base pairs), preferably about 100 base pairs to about 10,000 base pairs, more preferably about 500 base pairs to about 8,000 base pairs, even more preferably about 1,500 base pairs to about 6,000 base pairs, and most preferably about 2,000 to about 3,000 base pairs in length. The spacer element sequence can be coding or non-coding and native or non-native with respect to the adenoviral genome, but does not restore the replication-essential function to the deficient region. The spacer element preferably is located in the E4 region of the adenoviral genome. The use of a spacer in an adenoviral vector is described in U.S. Patent 5,851,806.

It has been observed that an at least E4-deficient adenoviral vector expresses a transgene at high levels for a limited amount of time *in vivo* and that persistence of expression of a transgene in an at least E4-deficient adenoviral vector can be modulated through the action of a trans-acting factor, such as HSV ICP0, Ad pTP, CMV-IE2, CMV-IE86, HIV tat, HTLV-tax, HBV-X, AAV Rep 78, the cellular factor from the U205 osteosarcoma cell line that functions like HSV ICP0, or the cellular factor in PC12 cells that is induced by nerve growth factor, among others, as described in for example, U.S. Patent 6,225,113, U.S. Patent Application Publication 2002/0031823 A1, and International Patent Application WO 00/34496. In view of the above, a multiply deficient adenoviral vector (e.g., the at least E4-deficient adenoviral vector) or a second expression vector desirably comprises a nucleic acid sequence encoding a trans-acting factor that modulates the persistence of expression of the nucleic acid sequence. Persistent expression of antigenic DNA can be desired when generating immune tolerance.

The adenoviral vector can be deficient in replication-essential gene functions of only the early regions of the adenoviral genome, only the late regions of the adenoviral genome, and both the early and late regions of the adenoviral genome. The adenoviral vector also can have essentially the entire adenoviral genome removed, in which case it is preferred that at least either the viral inverted terminal repeats (ITRs) and one or more promoters or the viral ITRs and a packaging signal are left intact (i.e., an adenoviral amplicon). In one embodiment, the adenoviral vector of the invention comprises an adenoviral genome that lacks native nucleic acid sequences which encode adenoviral proteins. Adenoviral genomic elements required for replication and packaging of the adenoviral genome into adenoviral capsid proteins can be retained. Minimal adenoviral vectors lacking adenoviral protein coding sequences are termed "helper-dependent" adenoviral vectors, and often require complementation by helper adenovirus for efficient propagation. Suitable replication-deficient adenoviral vectors, including multiply replication-deficient adenoviral vectors, are disclosed in U.S. Patents 5,837,511; 5,851,806; 5,994,106; 6,127,175; and 6,482,616; U.S. Patent Application Publications 2001/0043922 A1, 2002/0004040 A1, 2002/0031831 A1, and 2002/0110545 A1, and International Patent Applications WO 94/28152, WO 95/02697, WO 95/16772, WO 95/34671, WO 96/22378, WO 97/12986, WO 97/21826, and WO 03/022311. Ideally, the replication-deficient adenoviral vector is present in a composition, e.g., a pharmaceutical composition, substantially free of replication-competent adenovirus (RCA) contamination (e.g., the pharmaceutical composition comprises less than about 1% of RCA contamination). Most desirably, the composition is RCA-free. Adenoviral vector compositions and stocks that are RCA-free are described in U.S. Patent 5,944,106, U.S. Patent Application Publication 2002/0110545 A1, and International Patent Application WO 95/34671.

If the adenoviral vector is not replication-deficient, ideally the adenoviral vector is manipulated to limit replication of the vector to within a target tissue. For example, the adenoviral vector can be a conditionally-replicating adenoviral vector, which is engineered to replicate under conditions pre-determined by the practitioner. For example, replication-essential gene functions, e.g., gene functions encoded by the adenoviral early regions, can be operably linked to an inducible, repressible, or tissue-specific transcription control sequence, e.g., promoter. In this embodiment, replication requires the presence or absence of specific factors that interact with the transcription control sequence. In autoimmune disease treatment, it can be advantageous to control adenoviral vector replication in, for instance, lymph nodes, to obtain continual antigen production and control immune cell production. Conditionally-replicating adenoviral vectors are described further in U.S. Patent 5,998,205.

The adenoviral vector of the inventive method comprises an adenoviral fiber protein, wherein the amino acid sequence of the adenoviral fiber protein is 80% or more identical to that of a subgroup C adenoviral fiber protein. Coat proteins of the adenoviral vector, including the adenoviral vector fiber protein, can be modified to enhance or reduce immunogenicity, broaden or restrict tropism of the vector, and/or display one or more antigens on the viral surface, by removal and/or insertion of amino acids. For example, the adenoviral vector can comprise a chimeric coat protein comprising a non-native amino acid sequence that binds a substrate (i.e., a ligand). The non-native amino acid sequence of the chimeric adenoviral coat protein allows an adenoviral vector comprising the chimeric adenoviral coat protein to bind and, desirably, infect host cells not naturally infected by the corresponding adenovirus without the non-native amino acid sequence (i.e., host cells not infected by the corresponding wild-type adenovirus), to bind to host cells naturally infected by the corresponding adenovirus with greater affinity than the corresponding adenovirus without the non-native amino acid sequence, or to bind to particular target cells with greater affinity than non-target cells. By "preferentially binds" is meant that the non-native amino acid sequence binds a receptor, such as, for instance, αᵥβ₃ integrin, with at least about 3-fold greater affinity (e.g., at least about 5-fold, 10-fold, 15-fold, 20-fold, 25-fold, 35-fold, 45-fold, or 50-fold greater affinity) than the non-native ligand binds a different receptor, such as, for instance, αᵥβ₁ integrin.

Desirably, the adenoviral vector comprises a chimeric coat protein comprising a non-native amino acid sequence that confers to the chimeric coat protein the ability to bind to an immune cell more efficiently than a wild-type adenoviral coat protein. In particular, the adenoviral vector can comprise a chimeric adenoviral fiber protein comprising a non-native amino acid sequence which facilitates uptake of the adenoviral vector by immune cells, preferably antigen presenting cells, such as dendritic cells, monocytes, and macrophages. In a preferred embodiment, the adenoviral vector comprises a chimeric fiber protein comprising an amino acid sequence (e.g., a non-native amino acid sequence) comprising an RGD motif including, but not limited to, CRGDC (SEQ ID NO: 1), CXCRGDCXC (SEQ ID NO: 2), wherein X represents any amino acid, and CDCRGDCFC (SEQ ID NO: 3), which increases transduction efficiency of an adenoviral vector into dendritic cells. The RGD-motif, or any non-native amino acid sequence ligand, preferably is inserted into the adenoviral fiber knob region, ideally in an exposed loop of the adenoviral knob, such as the HI loop. A non-native amino acid sequence also can be appended to the C-terminus of the adenoviral fiber protein, optionally via a spacer sequence.

Where dendritic cells are the desired target cell, the non-native amino acid sequence can recognize a protein typically found on dendritic cell surfaces such as adhesion proteins, chemokine receptors, complement receptors, co-stimulation proteins, cytokine receptors, high level antigen presenting molecules, homing proteins, marker proteins, receptors for antigen uptake, signaling proteins, virus receptors, etc. Examples of such potential ligand-binding sites in dendritic cells include αᵥβ₃ integrins, αᵥβ₅ integrins, 2A1, 7-TM receptors, CD1, CD11a, CD11b, CD11c, CD21, CD24, CD32, CD4, CD40, CD44 variants, CD46, CD49d, CD50, CD54, CD58, CD64, ASGPR, CD80, CD83, CD86, E-cadherin, integrins, M342, MHC-I, MHC-II, MIDC-8, MMR, OX62, p200-MR6, p55, S 100, TNF-R, etc. Preferably, where dendritic cells are targeted, the ligand recognizes the CD40 cell surface protein, such as, for example, a CD-40 (bi)specific antibody fragment or a domain derived from the CD40L polypeptide.

Where macrophages are the desired target, the non-native amino acid sequence can recognize a protein typically found on macrophage cell surfaces, such as phosphatidylserine receptors, vitronectin receptors, integrins, adhesion receptors, receptors involved in signal transduction and/or inflammation, markers, receptors for induction of cytokines, or receptors up-regulated upon challenge by pathogens, members of the group B scavenger receptor cysteine-rich (SRCR) superfamily, sialic acid binding receptors, members of the Fc receptor family, B7-1 and B7-2 surface molecules, lymphocyte receptors, leukocyte receptors, antigen presenting molecules, and the like. Examples of suitable macrophage surface target proteins include, but are not limited to, heparin sulfate proteoglycans, αᵥβ₃ integrins, αᵥβ₅ integrins, B7-1, B7-2, CD11c, CD13, CD16, CD163, CD1a, CD22, CD23, CD29, Cd32, CD33, CD36, CD44, CD45, CD49e, CD52, CD53, CD54, CD71, CD87, CD9, CD98, Ig receptors, Fc receptor proteins (e.g., subtypes of Fcα, Fcγ, Fcε, etc.), folate receptor b, HLA Class I, Sialoadhesin, siglec-5, and the toll-like receptor-2 (TLR2).

Where B-cells are the desired target, the ligand can recognize a protein typically found on B-cell surfaces, such as integrins and other adhesion molecules, complement receptors, interleukin receptors, phagocyte receptors, immunoglobulin receptors, activation markers, transferrin receptors, members of the scavenger receptor cysteine-rich (SRCR) superfamily, growth factor receptors, selectins, MHC molecules, TNF-receptors, and TNF-R associated factors. Examples of typical B-cell surface proteins include β-glycan, B cell antigen receptor (BAC), B7-2, B-cell receptor (BCR), C3d receptor, CD1, CD18, CD19, CD20, CD21, CD22, CD23, CD35, CD40, CD5, CD6, CD69, CD69, CD71, CD79a/CD79b dimer, CD95, endoglin, Fas antigen, human Ig receptors, Fc receptor proteins (e.g., subtypes of Fca, Fcg, Fcε, etc.), IgM, gp200-MR6, Growth Hormone Receptor (GH-R), ICAM-1, ILT2, CD85, MHC class I and II molecules, transforming growth factor receptor (TGF-R), α₄β₇ integrin, and αᵥβ₃ integrin.

In another embodiment, the adenoviral vector comprises a chimeric virus coat protein not selective for a specific type of eukaryotic cell. The chimeric coat protein differs from a wild-type coat protein by an insertion of a non-native amino acid sequence into or in place of an internal coat protein sequence, or attachment of a non-native amino acid sequence to the N- or C- terminus of the coat protein. For example; a ligand comprising about five to about nine lysine residues (preferably seven lysine residues) is attached to the C-terminus of the adenoviral fiber protein via a non-coding spacer sequence. In this embodiment, the chimeric virus coat protein efficiently binds to a broader range of eukaryotic cells than a wild-type virus coat, such as described in U.S. Patent 6,465,253 and International Patent Application WO 97/20051. Such an adenoviral vector is preferred to ensure widespread production of the antigen.

The ability of an adenoviral vector to recognize a potential host cell can be modulated without genetic manipulation of the coat protein, i.e., through use of a bi-specific molecule. For instance, complexing an adenovirus with a bispecific molecule comprising a penton base-binding domain and a domain that selectively binds a particular cell surface binding site enables the targeting of the adenoviral vector to a particular cell type. Likewise, an antigen can be conjugated to the surface of the adenoviral particle through non-genetic means.

A non-native amino acid sequence can be conjugated to any of the adenoviral coat proteins to form a chimeric adenoviral coat protein. Therefore, for example, a non-native amino acid sequence can be conjugated to, inserted into, or attached to a fiber protein, a penton base protein, a hexon protein, proteins IX, VI, or IIIa, etc. The sequences of such proteins, and methods for employing them in recombinant proteins, are well known in the art (see, e.g., U.S. Patents 5,543,328; 5,559,099; 5,712,136; 5,731,190; 5,756,086; 5,770,442; 5,846,782; 5,962,311; 5,965,541; 5,846,782; 6,057,155; 6,127,525; 6,153,435; 6,329,190; 6,455,314; 6,465,253; 6,576,456; 6,649,407; 6,740,525, and International Patent Applications WO 96/07734, WO 96/26281, WO 97/20051, WO 98/07877, WO 98/07865, WO 98/40509, WO 98/54346, WO 00/15823, and WO 01/58940). The chimeric adenoviral coat protein can be generated using standard recombinant DNA techniques known in the art and described herein. Preferably, the nucleic acid sequence encoding the chimeric adenoviral coat protein is located within the adenoviral genome and is operably linked to a promoter that regulates expression of the coat protein in a wild-type adenovirus. Alternatively, the nucleic acid sequence encoding the chimeric adenoviral coat protein is located within the adenoviral genome and is part of an expression cassette which comprises the genetic elements required for efficient expression of the chimeric coat protein (e.g., a heterologous expression control sequence as described herein).

The coat protein portion of the chimeric adenovirus coat protein can be a full-length adenoviral coat protein to which the ligand domain is appended, or it can be truncated, e.g., internally or at the C- and/or N- terminus. However modified (including the presence of the non-native amino acid), the chimeric coat protein preferably is able to incorporate into an adenoviral capsid. Where the non-native amino acid sequence is attached to the fiber protein, preferably it does not disturb the interaction between viral proteins or fiber monomers. Thus, the non-native amino acid sequence preferably is not itself an oligomerization domain, as such can adversely interact with the trimerization domain of the adenovirus fiber. Preferably the non-native amino acid sequence is added to the virion protein, and is incorporated in such a manner as to be readily exposed to a substrate, cell surface-receptor, or immune cell (e.g., at the N- or C- terminus of the adenoviral protein, attached to a residue facing a substrate, positioned on a peptide spacer, etc.) to maximally expose the non-native amino acid sequence. Ideally, the non-native amino acid sequence is incorporated into an adenoviral fiber protein at the C-terminus of the fiber protein (and attached via a spacer) or incorporated into an exposed loop (e.g., the HI loop) of the fiber to create a chimeric coat protein. Where the non-native amino acid sequence is attached to or replaces a portion of the penton base, preferably it is within the hypervariable regions to ensure that it contacts the substrate. Where the non-native amino acid sequence is attached to the hexon, preferably it is within a hypervariable region (Miksza et al, J. Virol., 70(3), 1836-44 (1996)). Where the non-native amino acid is attached to or replaces a portion of pIX, preferably it is within the C-terminus of pIX. Use of a spacer sequence to extend the non-native amino acid sequence away from the surface of the adenoviral particle can be advantageous in that the non-native amino acid sequence can be more available for binding to a receptor, and any steric interactions between the non-native amino acid sequence and the adenoviral fiber monomers can be reduced.

While a non-subgroup C adenoviral vector preferably is employed in the invention to evade existing immunity and increase persistence of the vector in the mammal, the adenoviral vector comprises an adenoviral fiber that enters host cells via CAR binding (e.g., a subgroup C adenoviral fiber protein) to provoke an immune response against the encoded antigen. To this end, the adenoviral fiber protein can comprise a non-native amino acid sequence resulting in increased immunogenicity (antigenicity) of the adenoviral vector compared to an adenoviral vector that is the same except for the presence of the non-native amino acid sequence in the adenoviral fiber protein. For example, a non-native amino acid sequence comprising an RGD motif can be inserted into the adenoviral fiber protein, which enhances the immunogenicity of the protein in a mammal. For example, a non-native amino acid sequence comprising CRGDC (SEQ ID NO: 1) can be inserted into or appended to the adenoviral fiber protein. Other suitable RGD-containing non-native amino acid sequences include, but are not limited to, CXCRGDCXC (SEQ ID NO: 2), wherein X can be any amino acid, and CDCRGDCFC (SEQ ID NO: 3). The adenoviral fiber protein (or other adenoviral coat protein) also can be modified by non-genetic means to increase the adjuvant effect of the viral particle.

If desired, native binding of adenoviral coat proteins to a cell surface receptor can be interrupted, thereby reducing transduction of host cells. In this embodiment, the native binding sites located on adenoviral coat proteins which mediate cell entry, e.g., the fiber and/or penton base, are absent or disrupted. Two or more of the adenoviral coat proteins are believed to mediate attachment to cell surfaces (e.g., the fiber and penton base). Any suitable technique for altering native binding to a host cell (e.g., binding to coxsackievirus and adenovirus receptor (CAR)) can be employed. For example, exploiting differing fiber lengths to ablate native binding to cells can be accomplished via the addition of a binding sequence to the penton base or fiber knob. This addition can be done either directly or indirectly via a bispecific or multispecific binding sequence. Alternatively, the adenoviral fiber protein can be modified to reduce the number of amino acids in the fiber shaft, thereby creating a "short-shafted" fiber (as described in, for example, U.S. Patent 5,962,311).

Alternatively, the nucleic acid residues associated with native substrate binding can be mutated (see, e.g., International Patent Application WO 00/15823; Einfeld et al., J. Virol., 75(23), 11284-11291 (2001); and van Beusechem et al., J. Virol., 76(6), 2753-2762 (2002)) such that the adenoviral vector incorporating the mutated nucleic acid residues is less able to bind its native substrate. For example, adenovirus serotypes 2 and 5 transduce cells via binding of the adenoviral fiber protein to CAR and binding of penton proteins to integrins located on the cell surface. The adenoviral vector can lack native receptor binding to, for example, CAR and/or exhibit reduced native binding to integrins by removing or disrupting the native CAR and/or integrin binding sites (e.g., the RGD sequence located in the adenoviral penton base). Likewise, the native binding of subgroup B2 adenoviral fiber proteins to CD46 can be reduced or ablated. Removal or mutation of native binding sites can significantly reduce native binding (i.e., a modified chimeric adenoviral fiber protein binds a native cell surface receptor with at least about 5-fold, 10-fold, 20-fold, 30-fold, 50-fold, or 100-fold less affinity than a non-modified adenoviral fiber protein of the same serotype).

The adenoviral vector preferably comprises a chimeric coat protein (e.g., a chimeric adenoviral fiber, hexon, or pIX protein) comprising an amino acid sequence encoding an antigen. Antigens are presented to immune effector cells by antigen presenting cells in two recognizable formats. Antigens captured and taken up by antigen presenting cells are processed and presented on the cell surface by major histocompatibility complex II (MHC II) complexes. MHC II-presented antigens stimulate helper and, indirectly, cytotoxic T-cells, as well as the B-cell-mediated humoral immune response. The antigen encoded by the nucleic acid sequence of the adenoviral vector is expressed within the cell and is presented on the antigen presenting cell surface associated with MHC I, which activates cytotoxic T-cells. Thus, the invention envisions a two-pronged method of presenting antigens to the immune system by providing antigenic epitopes on the viral surface and producing antigens intracellularly, resulting in an enhanced immune response compared to that generated by MHC I or MHC II antigen presentation alone. Methods of eliciting an MHC I and MHC II-mediated immune response are further described in International Patent Application WO 01/58478.

Suitable modifications to an adenoviral vector are described in U.S. Patents 5,543,328; 5,559,099; 5,712,136; 5,731,190; 5,756,086; 5,770,442; 5,846,782; 5,871,727; 5,885,808; 5,922,315; 5,962,311; 5,965,541; 6,057,155; 6,127,525; 6,153,435; 6,329,190; 6,455,314; 6,465,253; 6,576,456; 6,649,407; and 6,740,525; U.S. Patent Application Publications 2001/0047081 A1, 2002/0099024 A1, 2002/0151027 A1, 2003/0022355 A1, and 2003/0099619 A1, and International Patent Applications WO 96/07734, WO 96/26281, WO 97/20051, WO 98/07865, WO 98/07877, WO 98/40509, WO 98/54346, WO 00/15823, WO 01/58940, and WO 01/92549.

The invention further provides an adenoviral vector comprising a nucleic acid sequence encoding an adenoviral pIX protein operably linked to a heterologous expression control sequence. The nucleic acid sequence preferably encodes a wild-type adenoviral pIX protein, some examples of which are set forth at SEQ ID NOs: 4-10; however, the invention is not limited to these exemplary sequences. Indeed, genetic sequences can vary between different strains, and this natural scope of allelic variation is included within the scope of the invention. Thus, the nucleic acid sequence preferably encodes a pIX protein that is at least about 75% homologous to at least about 15 contiguous amino acids of one of SEQ ID NOs: 4-10 and preferably is at least about 80% homologous to at least about 15 contiguous amino acids of one of SEQ ID NOs: 4-10 (e.g., at least about 85% homologous to at least about 15 contiguous amino acids of one of SEQ ID NOs: 4-10); more preferably the nucleic acid sequence encodes a pIX protein that is at least about 90% homologous to at least about 15 contiguous amino acids of one of SEQ ID NOs: 4-10 (such as at least about 95% homologous to at least about 15 contiguous amino acids of one of SEQ ID NOs: 4-10), and most preferably the nucleic acid sequence encodes a pIX protein that is at least about 97% homologous to at least about 15 contiguous amino acids of one of SEQ ID NOs: 4-10. Preferably, the homology extends to at least 25 contiguous amino acids, such as at least about 50 contiguous amino acids. Determining the degree of homology, including the possibility for gaps, can be accomplished using any method known to those of skill in the art (e.g., Clustal or J. Hein method using PAM100 or PAM 250 residue weight table, BLASTp, etc.). The nucleic acid sequence encoding the pIX protein can be obtained from any source, e.g., isolated from nature, synthetically generated, isolated from a genetically engineered organism, and the like. While it is preferred that the nucleic acid sequence encoding the adenoviral pIX protein is located in the adenoviral genome, the nucleic acid sequence can be introduced into a suitable complementing cell line (e.g., on a plasmid) together with the adenoviral vector.

The nucleic acid sequence encoding an adenoviral pIX protein is operably linked to a heterologous expression control sequence. An "expression control sequence" is any nucleic acid sequence that promotes, enhances, or controls expression (typically and preferably transcription) of another nucleic acid sequence. Typically and preferably, the expression control sequence comprises double-stranded DNA. Alternatively, the expression control sequence comprises double-stranded RNA, an RNA-DNA hybrid, or synthetically generated nucleotides. Preferably, the expression control sequence comprises, consists of, or consists essentially of a nucleic acid sequence that functions to direct the binding of RNA polymerase and thereby promotes transcription of the operably linked nucleic acid sequence (e.g., a promoter sequence or portion thereof). Alternatively, the expression control sequence comprises, consists of, or consists essentially of a nucleic acid sequence that functions to direct splicing of the operably linked nucleic acid sequence into a mRNA molecule transcribed from an upstream expression control sequence (e.g., a splice acceptor sequence). A nucleic acid sequence is "operably linked" to an expression control sequence when the expression control sequence is capable of promoting, enhancing, or controlling expression (typically and preferably transcription) of that nucleic acid sequence.

The expression control sequence is "heterologous" in that the expression control sequence is different from, or in a different position relative to, the promoter operably linked to the nucleic acid sequence encoding the pIX protein in a wild-type adenovirus. Preferably, the heterologous expression control sequence is not obtained from, derived from, or based upon a naturally occurring adenoviral pIX promoter. An expression control sequence is "obtained" from a source when it is isolated from that source. An expression control sequence is "derived" from a source when it comprises a sequence isolated from a source but modified in any suitable manner (e.g., by mutation or other modification to the sequence). An expression control sequence is "based upon" a source when its sequence is highly homologous to the source but obtained through synthetic procedures (e.g., polynucleotide synthesis, directed evolution, etc.). By "naturally occurring" is meant that the expression control sequence is encoded by a nucleic acid sequence that can be found in nature and has not been synthetically modified. Notwithstanding the foregoing, however, the heterologous expression control sequence can be naturally found in adenovirus, but located at a normative position with respect to the nucleic acid sequence encoding the pIX protein. For example, in one embodiment, an E1/E4-deficient adenoviral vector can comprise an expression cassette (e.g., a nucleic acid sequence encoding an antigen) positioned in the deleted E4 region of the adenoviral genome. Therefore, as a result of the deletion of the E1 region, the E1A promoter is positioned directly upstream of the nucleic acid sequence encoding pIX, such that pIX expression is regulated by the E1A promoter. Operable linkage of the heterologous expression control sequence to the pIX-encoding nucleic acid sequence can be performed using any recombinant DNA technique known in the art, such as those described in Sambrook et al., *supra.*

The heterologous expression control sequence preferably is a heterologous promoter. A "promoter" is a DNA sequence that directs the binding of RNA polymerase and thereby promotes RNA synthesis. Any promoter (i.e., whether isolated from nature or produced by recombinant DNA or synthetic techniques) can be used in connection with the invention to provide for transcription of the nucleic acid sequence. The promoter preferably is capable of directing transcription in a eukaryotic (desirably mammalian) cell. The functioning of the promoter can be altered by the presence of one or more enhancers (e.g., the CMV immediate early enhancer) and/or silencers.

The invention preferably employs a viral promoter. Suitable viral promoters are known in the art and include, for instance, cytomegalovirus (CMV) promoters, such as the CMV immediate-early promoter, promoters derived from human immunodeficiency virus (HIV), such as the HIV long terminal repeat promoter, Rous sarcoma virus (RSV) promoters, such as the RSV long terminal repeat, mouse mammary tumor virus (MMTV) promoters, HSV promoters, such as the Lap2 promoter or the herpes thymidine kinase promoter (Wagner et al., Proc. Natl. Acad. Sci., 78, 144-145 (1981)), promoters derived from SV40 or Epstein Barr virus, an adeno-associated viral promoter, such as the p5 promoter, and the like. Preferably, the viral promoter is an adeno-associated virus p5 promoter of SEQ ID NO: 11.

The heterologous promoter need not be a viral promoter. For example, the heterologous promoter can be a cellular promoter, i.e., a promoter that drives expression of a cellular protein. Preferred cellular promoters for use in the invention will depend on the desired expression profile to produce the protein(s) encoded by the nucleic acid sequence. In one aspect, the cellular promoter is preferably a constitutive promoter that works in a variety of cell types. Suitable constitutive promoters can drive expression of genes encoding transcription factors, housekeeping genes, or structural genes common to eukaryotic cells. For example, the Ying Yang 1 (YY1) transcription factor (also referred to as NMP-1, NF-E1, and UCRBP) is a ubiquitous nuclear transcription factor that is an intrinsic component of the nuclear matrix (Guo et al., PNAS, 92, 10526-10530 (1995)). JEM-1 (also known as HGMW and BLZF-1; Tong et al., Leukemia, 12(11), 1733-1740 (1998), and Tong et al., Genomics, 69(3), 380-390 (2000)), a ubiquitin promoter, specifically UbC (Marinovic et al., J. Biol. Chem., 277(19), 16673-16681 (2002)), a β-actin promoter, such as that derived from chicken, and the like are appropriate for use in the inventive method.

Many of the above-described promoters are constitutive promoters. Instead of being a constitutive promoter, the heterologous promoter can be a regulatable promoter, i.e., a promoter that is up- and/or down-regulated in response to appropriate signals. The use of a regulatable promoter or expression control sequence is particularly applicable to DNA vaccine development as antigenic proteins, including viral and parasite antigens, frequently are toxic to complementing cell lines. In one embodiment, the regulatory sequences operably linked to the pIX-encoding nucleic acid sequence include components of the tetracycline expression system, e.g., tet operator sites. For instance, the pIX-encoding nucleic acid sequence is operably linked to a promoter which is operably linked to one or more tet operator sites. An adenoviral vector comprising such an expression cassette can be propagated in a complementing cell line, such as 293-ORF6 described in, for example, U.S. Patent 5,994,106 and International Patent Application WO 95/34671, which comprises a nucleic acid sequence encoding a tet repressor protein. By producing the tet repressor protein in the complementing cell line, pIX production is inhibited and propagation proceeds without any associated cell toxicity. Suitable heterologous regulatable promoter systems also include, but are not limited to, the IL-8 promoter, the metallothionine inducible promoter system, the bacterial lacZYA expression system, and the T7 polymerase system. Further, promoters that are selectively activated at different developmental stages (e.g., globin genes are differentially transcribed from globin-associated promoters in embryos and adults) can be employed. The heterologous promoter sequence can contain at least one heterologous regulatory sequence responsive to regulation by an exogenous agent. The regulatory sequences are preferably responsive to exogenous agents such as, but not limited to, drugs, hormones, or other gene products.

Alternatively, the heterologous expression control sequence can be a heterologous enhancer. An enhancer is any *cis*-acting polynucleotide sequence that promotes, induces, or otherwise controls (e.g., inhibits) expression (preferably transcription) of one or more operatively linked nucleic acid sequences. An enhancer that inhibits transcription also is termed a "silencer." The enhancer can function in either a direct or reverse orientation with respect to the nucleic acid sequence (e.g., from a position "downstream" of the operatively linked nucleic acid sequence) and over a relatively large distance (e.g., several kilobases (kb)) from an operatively linked nucleic acid sequence. Accordingly, the heterologous enhancer can be any nucleic acid sequence that can function to induce, promote, or control expression in either orientation and/or at various distances from the operably linked nucleic acid sequence. In contrast, a promoter will operate in a sequence specific manner, typically in the same orientation with and upstream from the nucleic acid sequence, and in a more localized manner.

The heterologous expression control sequence can comprise either a heterologous promoter or a heterologous enhancer, or a hybrid expression control sequence can be constructed which comprises both a heterologous promoter and a heterologous enhancer, or functional portions thereof. In accordance with the invention, a "functional portion" is any portion of an expression control sequence that measurably promotes, enhances, or controls expression (typically transcription) of an operatively linked nucleic acid. Such regulation of expression can be measured via RNA or protein detection by any suitable technique, and several such techniques are known in the art. Examples of such techniques include Northern analysis (see, e.g., Sambrook et al., *supra,* and McMaster and Carmichael, PNAS, 74, 4835-4838 (1977)), RT-PCR (see, e.g., U.S. Patent 5,601,820, and Zaheer et al., Neurochem Res., 20, 1457-1463 (1995)), *in situ* hybridization methods (see, e.g., U.S. Patents 5,750,340 and 5,506,098), antibody-mediated techniques (see, e.g., U.S. Patents 4,367,110, 4,452,901, and 6,054,467), and promoter assays utilizing reporter gene systems such as the luciferase gene (see, e.g., Taira et al., Gene, 263, 285-292 (2001)). Eukaryotic expression systems in general are further detailed in Sambrook et al., *supra.*

In another embodiment of the invention, the heterologous expression control sequence can be an anti-repressor nucleic acid sequence. An "anti-repressor" is a cis-acting nucleic acid sequence that functions to counteract repression of gene expression, but is not itself a promoter sequence. Any suitable anti-repressor sequence can be used in the context of the invention. Preferably, the anti-repressor nucleic acid sequence is obtained from, based upon, or derived from a bacterium or a mammal (see, e.g., Kwaks et al., Nat. Biotechnol., 21(5), 553-8 (2003)).

Whatever heterologous expression control sequence is chosen, it preferably deregulates expression of the nucleic acid sequence encoding pIX from the promoter operably linked to the nucleic acid sequence encoding the pIX protein in a wild-type adenovirus. As discussed herein, adenoviral vectors frequently are made replication deficient by deletion of all or part of the E1 region of the adenoviral genome. Deletion of the E1 region, however, has been shown to downregulate pIX expression, resulting in adenoviral particles that are unstable at high temperatures (i.e., "thermolabile") (see, e.g., Ghosh-Choudhury et al., *supra*). Thermolabile adenoviral vectors cannot package full-length genomes (see, e.g., Caravokyri et al., *supra*). It has been found that altering pIX expression by releasing the pIX gene from its endogenous expression control mechanisms produces adenoviral vectors that are more stable at high temperatures (i.e., "thermostable"), as compared to adenoviral vectors deficient in pIX protein (e.g., as a result of deletion of the E1 region). As such, the thermostability conferred by restoration of pIX expression increases the efficiency of virus rescue from plasmid DNA. Thermostability also confers enhanced stability of adenoviral genomes, thereby increasing the packaging capacity of the adenoviral vector.

The invention further provides a method of enhancing the stability and/or packaging capacity of an adenoviral vector. The method comprises: (a) introducing an adenoviral vector comprising a nucleic acid sequence encoding an adenoviral pIX protein operably linked to a heterologous expression control sequence into a cell, and propagating the adenoviral vector, wherein the stability and/or packaging capacity of the adenoviral vector is enhanced as compared to an adenoviral vector that lacks a pIX-encoding nucleic acid sequence operably linked to a heterologous expression control sequence. The stability of the adenoviral vector can be assayed using any suitable method, such as the thermostability assay described in the Examples. In a preferred embodiment of the inventive method, the packaging capacity of the adenoviral vector is enhanced such that it is about 95% or more (e.g., about 95%, about 97%, or about 98%) of a corresponding wild-type adenovirus genome. More preferably, the packaging capacity of the adenoviral vector is about 99% (e.g., about 99%, about 99.5%, or about 99.8%) or more of a corresponding wild-type adenovirus genome. Most preferably, the packaging capacity of the adenoviral vector is about 100% or more (e.g., about 100.5%, about 105%, or about 108%) of a corresponding wild-type adenovirus genome.

While the stability and/or packaging efficiency of the adenoviral vector can be enhanced by operably linking a nucleic acid sequence encoding pIX to a heterologous expression control sequence, adenoviral vector stability and/or packaging efficiency also can be enhanced by modifying the nucleic acid sequence encoding pIX, such that the activity of the pIX protein is increased as compared to a pIX protein encoded by an unmodified nucleic acid sequence. In this embodiment, the nucleic acid sequence encoding pIX is operably linked to a wild-type pIX promoter. The nucleic acid sequence preferably is mutated such that the pIX protein contains a deletion, substitution, or addition of one or more amino acids. The pIX-encoding nucleic acid sequence can be mutated in any suitable manner, so long as the activity of the pIX protein is increased as compared to a pIX protein encoded by an unmodified nucleic acid sequence. The activity of the pIX protein can be measured using any suitable method, including the virus viability and stability assays described in the Examples.

The adenoviral vector of the invention comprises a nucleic acid sequence encoding an antigen which is expressed in the mammal to induce an immune response. An "antigen" is a molecule that triggers an immune response in a mammal. An "immune response" can entail, for example, antibody production and/or the activation of immune effector cells. An antigen in the context of the invention can comprise any subunit of any proteinaceous molecule, including a protein or peptide of viral, bacterial, parasitic, fungal, protozoan, prion, cellular, or extracellular origin, which ideally provokes an immune response in mammal, preferably leading to protective immunity. The antigen also can be a self antigen, i.e., an autologous protein which the body has mistakenly identified as a foreign invader.

In one embodiment, the antigen is a viral antigen. The viral antigen can be isolated from any virus including, but not limited to, a virus from any of the following viral families: *Arenaviridae, Arterivirus, Astroviridae, Baculoviridae, Badnavirus, Barnaviridae, Birnaviridae, Bromoviridae, Bunyaviridae, Caliciviridae, Capillovirus, Carlavirus, Caulimovirus, Circoviridae, Closterovirus, Comoviridae, Coronaviridae* (e.g., Coronavirus, such as severe acute respiratory syndrome (SARS) virus), *Corticoviridae, Cystoviridae, Deltavirus, Dianthovirus, Enamovirus, Filoviridae* (e.g., Marburg virus and Ebola virus (e.g., Zaire, Reston, Ivory Coast, or Sudan strain)), *Flaviviridae,* (e.g., Hepatitis C virus, Dengue virus 1, Dengue virus 2, Dengue virus 3, and Dengue virus 4), *Hepadnaviridae* (e.g., Hepatitis B virus), *Herpesviridae* (e.g., Human herpesvirus 1, 3, 4, 5, and 6, and Cytomegalovirus), *Hypoviridae, Iridoviridae, Leviviridae, Lipothrixviridae, Microviridae, Orthomyxoviridae* (e.g., Influenzavirus A and B), *Papovaviridae, Paramyxoviridae* (e.g., measles, mumps, and human respiratory syncytial virus), *Parvoviridae, Picornaviridae* (e.g., poliovirus, rhinovirus, hepatovirus, and aphthovirus), *Poxviridae* (e.g., vaccinia virus), *Reoviridae* (e.g., rotavirus), *Retroviridae* (e.g., lentivirus, such as human immunodeficiency virus (HIV) 1 and HIV 2), *Rhabdoviridae,* and *Totiviridae.* Preferably, at least one antigen of the inventive method is a retroviral antigen. The retroviral antigen can be, for example, an HIV antigen, such as all or part of the gag, env, or pol proteins. Any clade of HIV is appropriate for antigen selection, including clades A, B, C, MN, and the like. Also preferably, at least one antigen encoded by the adenoviral vector is a coronavirus antigen, such as a SARS virus antigen. Suitable SARS virus antigens for the inventive method include, for example, all or part of the E protein, the M protein, and the spike protein of the SARS virus. Suitable viral antigens also include all or part of Dengue protein M, Dengue protein E, Dengue D1NS1, Dengue D1NS2, and Dengue D1NS3. The antigenic peptides specifically recited herein are merely exemplary as any viral protein can be used in the context of the invention.

The antigen can be a parasite antigen such as, but not limited to, a *Sporozoan* antigen. For example, the nucleic acid sequence can encode a *Plasmodian* antigen, such as all or part of a Circumsporozoite protein, a Sporozoite surface protein, a liver stage antigen, an apical membrane associated protein, or a Merozoite surface protein.

Alternatively or in addition, at least one antigen encoded by the adenoviral vector is a bacterial antigen. The antigen can originate from any bacterium including, but not limited to, *Actinomyces, Anabaena, Bacillus, Bacteroides, Bdellovibrio, Caulobacter, Chlamydia, Chlorobium, Chromatium, Clostridium, Cytophaga, Deinococcus, Escherichia, Halobacterium, Heliobacter, Hyphomicrobium, Methanobacterium, Micrococcus, Myobacterium, Mycoplasma, Myxococcus, Neisseria, Nitrobacter, Oscillatoria, Prochloron, Proteus, Pseudomonas, Phodospirillum, Rickettsia, Salmonella, Shigella, Spirillum, Spirochaeta, Staphylococcus, Streptococcus, Streptomyces, Sulfolobus, Thermoplasma, Thiobacillus,* and *Treponema.* In a preferred embodiment, at least one antigen encoded by the nucleic acid sequence is a *Pseudomonas* antigen or a *Heliobacter* antigen.

It will be appreciated that an entire, intact viral or bacterial protein is not required to produce an immune response. Indeed, most antigenic epitopes are relatively small in size and, therefore, protein fragments can be sufficient for exposure to the immune system of the mammal. In addition, a fusion protein can be generated between two or more antigenic epitopes of one or more antigens. For example, all or part ofHIV gp120 or gp 160 can be fused to all or part of the HIV pol protein to generate a more complete immune response against the HIV pathogen compared to that generated by a single epitope. Delivery of fusion proteins via adenoviral vector to a mammal allows exposure of an immune system to multiple antigens and, accordingly, enables a single vaccine composition to provide immunity against multiple pathogens.

The nucleic acid sequence encoding the antigen is not limited to a type of nucleic acid sequence or any particular origin. The nucleic acid sequence can be recombinant DNA, can be genomic DNA, can be obtained from a DNA library of potential antigenic epitopes, or can be synthetically generated. The nucleic acid sequence can be present as part of an expression cassette, which additionally comprises the genetic elements required for efficient expression of the nucleic acid sequence and production of the encoded antigen. Ideally, the antigen-encoding nucleic acid sequence is operably linked to a promoter and a polyadenylation sequence as described herein. A promoter can be selected for use in the method of the invention by matching its particular pattern of activity with the desired pattern and level of expression of the antigen(s). For example, the adenoviral vector can comprise two or more nucleic acid sequences that encode different antigens and are operably linked to different promoters displaying distinct expression profiles. For example, a first promoter is selected to mediate an initial peak of antigen production, thereby priming the immune system against an encoded antigen. A second promoter is selected to drive production of the same or different antigen such that expression peaks several days after that of the first promoter, thereby "boosting" the immune system against the antigen. Alternatively, a hybrid promoter can be constructed which combines the desirable aspects of multiple promoters. For example, a CMV-RSV hybrid promoter combining the CMV promoter's initial rush of activity with the RSV promoter's high maintenance level of activity is especially preferred for use in many embodiments of the inventive method. In that antigens can be toxic to eukaryotic cells, it may be advantageous to modify the promoter to decrease activity in complementing cell lines used to propagate the adenoviral vector.

To optimize protein production, preferably the nucleic acid sequence encoding the antigen further comprises a polyadenylation site following the coding sequence of the nucleic acid sequence. Any suitable polyadenylation sequence can be used, including a synthetic optimized sequence, as well as the polyadenylation sequence of BGH (Bovine Growth Hormone), polyoma virus, TK (Thymidine Kinase), EBV (Epstein Barr Virus), and the papillomaviruses, including human papillomaviruses and BPV (Bovine Papilloma Virus). A preferred polyadenylation sequence is the SV40 (Human Sarcoma Virus-40) polyadenylation sequence. Also, preferably all the proper transcription signals (and translation signals, where appropriate) are correctly arranged such that the nucleic acid sequence is properly expressed in the cells into which it is introduced. If desired, the nucleic acid sequence also can incorporate splice sites (i.e., splice acceptor and splice donor sites) to facilitate mRNA production.

If the antigen-encoding nucleic acid sequence encodes a processed or secreted protein or peptide, or a protein that acts intracellularly, preferably the antigen-encoding nucleic acid sequence further comprises the appropriate sequences for processing, secretion, intracellular localization, and the like. The antigen-encoding nucleic acid sequence can be operably linked to a signal sequence, which targets a protein to cellular machinery for secretion. Appropriate signal sequences include, but are not limited to, leader sequences for immunoglobulin heavy chains and cytokines, (see, for example, Ladunga, Current Opinions in Biotechnology, 11, 13-18 (2000)). Other protein modifications can be required to secrete a protein from a host cell, which can be determined using routine laboratory techniques. Preparing expression constructs encoding antigens and signal sequences is further described in, for example, U.S. Patent 6,500,641. Methods of secreting non-secretable proteins are further described in, for example, U.S. Patent 6,472,176, and International Patent Application WO 02/48377.

The antigen protein encoded by the nucleic acid sequence of the adenoviral vector also can be modified to attach or incorporate the antigen on the host cell surface. In this respect, the antigen can comprise a membrane anchor, such as a gpi-anchor, for conjugation onto the cell surface. A transmembrane domain can be fused to the antigen to incorporate a terminus of the antigen protein into the cell membrane. Other strategies for displaying peptides on a cell surface are known in the art and are appropriate for use in the context of the invention.

In the method of the invention, the adenoviral vector preferably is administered to a mammal (e.g., a human), wherein the nucleic acid sequence encoding the antigen is expressed to induce an immune response against the antigen. The immune response can be a humoral immune response, a cell-mediated immune response, or, desirably, a combination of humoral and cell-mediated immunity. Ideally, the immune response provides protection upon subsequent challenge with the pathogen comprising the antigen. However, protective immunity is not required in the context of the invention. Administration of the inventive adenoviral vector also can be used to create immune tolerance to lessen the severity of, for example, autoimmune diseases such as lupus, psoriasis, and arthritis. The inventive method further can be used for antibody production and harvesting.

To enhance the immune response generated against the antigen, the adenoviral vector, or a different gene transfer vector administered to the mammal, can comprise a nucleic acid sequence that encodes an immune stimulator, such as a cytokine, a chemokine, or a chaperone. Cytokines include, for example, Macrophage Colony Stimulating Factor (e.g., GM-CSF), Interferon Alpha (IFN-α), Interferon Beta (IFN-β), Interferon Gamma (IFN-γ), interleukins (IL-1, IL-2, IL-4, IL-5, IL-6, IL-8, IL-10, IL-12, IL-13, IL-15, IL-16, and IL-18), the TNF family of proteins, Intercellular Adhesion Molecule-1 (ICAM-1), Lymphocyte Function-Associated antigen-3 (LFA-3), B7-1, B7-2, FMS-related tyrosine kinase 3 ligand, (Flt3L), vasoactive intestinal peptide (VIP), and CD40 ligand. Chemokines include, for example, B Cell-Attracting chemokine-1 (BCA-1), Fractalkine, Melanoma Growth Stimulatory Activity protein (MGSA), Hemofiltrate CC chemokine 1 (HCC-1), Interleukin 8 (IL8), Interferon-stimulated T-cell alpha chemoattractant (I-TAC), Lymphotactin, Monocyte Chemotactic Protein 1 (MCP-1), Monocyte Chemotactic Protein 3 (MCP-3), Monocyte Chemotactic Protein 4 (MCP-4), Macrophage-Derived Chemokine (MDC), a macrophage inflammatory protein (MIP), Platelet Factor 4 (PF4), RANTES, BRAK, eotaxin, exodus 1-3, and the like. Chaperones include, for example, the heat shock proteins Hsp170, Hsc70, and Hsp40. Cytokines and chemokines are generally described in the art, including the Invivogen catalog (2002), San Diego, CA.

The invention can comprise administering multiple adenoviral vectors to the mammal, each adenoviral vector comprising one or more nucleic acid sequences encoding one or more antigens and/or immunomodulators. If the adenoviral vector comprises more than one exogenous nucleic acid sequence, two or more nucleic acid sequences can be operably linked to the same promoter (e.g., to form a bicistronic sequence), two or more nucleic acid sequences can be operably linked to separate promoters of the same type (e.g., the CMV promoter), or two or more nucleic acid sequences can be operably linked to separate and different promoters (e.g., the CMV promoter and β-actin promoter). The multiple adenoviral vectors can include two or more adenoviral vector constructs encoding different antigens, different epitopes of the same antigenic protein, the same antigenic protein derived from different species or clades of microorganism, antigens from different microorganisms, and the like. In will be appreciated that, in some embodiments, administering a "cocktail" of adenoviral vectors encoding different antigens or different epitopes of the same antigen can provide a more effective immune response than administering a single adenoviral vector clone to a mammal.

Likewise, administering the adenoviral vector encoding an antigen can be one component of a multistep regimen for inducing an immune response in a mammal. In particular, the inventive method can represent one arm of a prime and boost immunization regimen. The inventive method, therefore, can comprise administering to the mammal a priming gene transfer vector comprising a nucleic acid sequence encoding at least one antigen prior to administering the adenoviral vector. The antigen encoded by the priming gene transfer vector can be the same or different from the antigen of the adenoviral vector. The adenoviral vector is then administered to boost the immune response to a given pathogen. More than one boosting composition comprising the adenoviral vector can be provided in any suitable timeframe (e.g., at least about 1 week, 2 weeks, 4 weeks, 8 weeks, 12 weeks, 16 weeks, or more following priming) to maintain immunity.

Any gene transfer vector can be employed as a priming gene transfer vector, including, but not limited to, a plasmid, a retrovirus, an adeno-associated virus, a vaccinia virus, a herpesvirus, or an adenovirus. Ideally, the priming gene transfer vector is a plasmid or an adenoviral vector. Alternatively, an immune response can be primed or boosted by administration of the antigen itself, e.g., an antigenic protein, inactivated pathogen, and the like.

Any route of administration can be used to deliver the adenoviral vector to the mammal. Indeed, although more than one route can be used to administer the adenoviral vector, a particular route can provide a more immediate and more effective reaction than another route. Preferably, the adenoviral vector is administered via intramuscular injection. A dose of adenoviral vector also can be applied or instilled into body cavities, absorbed through the skin (e.g., via a transdermal patch), inhaled, ingested, topically applied to tissue, or administered parenterally via, for instance, intravenous, peritoneal, or intraarterial administration.

The adenoviral vector can be administered in or on a device that allows controlled or sustained release, such as a sponge, biocompatible meshwork, mechanical reservoir, or mechanical implant. Implants (see, e.g., U.S. Patent 5,443,505), devices (see, e.g., U.S. Patent 4,863,457), such as an implantable device, e.g., a mechanical reservoir or an implant or a device comprised of a polymeric composition, are particularly useful for administration of the adenoviral vector. The adenoviral vector also can be administered in the form of sustained-release formulations (see, e.g., U.S. Patent 5,378,475) comprising, for example, gel foam, hyaluronic acid, gelatin, chondroitin sulfate, a polyphosphoester, such as bis-2-hydroxyethyl-terephthalate (BHET), and/or a polylactic-glycolic acid.

The dose of adenoviral vector administered to the mammal will depend on a number of factors, including the size of a target tissue, the extent of any side-effects, the particular route of administration, and the like. The dose ideally comprises an "effective amount" of adenoviral vector, i.e., a dose of adenoviral vector which provokes a desired immune response in the mammal. The desired immune response can entail production of antibodies, protection upon subsequent challenge, immune tolerance, immune cell activation, and the like. Desirably, a single dose of adenoviral vector comprises at least about 1x10⁵ particles (which also is referred to as particle units) of the adenoviral vector. The dose preferably is at least about 1x10⁶particles (e.g., about 1x10⁶-1x10¹² particles), more preferably at least about 1x10⁷ particles, more preferably at least about 1x10⁸ particles (e.g., about 1x10⁸-1x10¹¹ particles), and most preferably at least about 1x10⁹ particles (e.g., about 1x10⁹-1x10¹⁰ particles) of the adenoviral vector. Alternatively, the dose comprises no more than about 1x10¹⁴ particles, preferably no more than about 1x10¹³ particles, even more preferably no more than about 1x10¹² particles, even more preferably no more than about 1x10¹¹ particles, and most preferably no more than about 1x10¹⁰ particles (e.g., no more than about 1x10⁹ particles). In other words, a single dose of adenoviral vector can comprise, for example, about 1x10⁶ particle units (pu), 2x10⁶ pu, 4x10⁶ pu, 1x10⁷ pu, 2x10⁷ pu, 4x10⁷ pu, 1x10⁸ pu, 2x10⁸ pu, 4x10⁸ pu, 1x10⁹ pu, 2x10⁹ pu, 4x10⁹ pu, 1x10¹⁰ pu, 2x10¹⁰ pu, 4x10¹⁰ pu, 1x10¹¹ pu, 2x10¹¹ pu, 4x10¹¹ pu, 1x10¹² pu, 2x10¹² pu, or 4x10¹² pu of the adenoviral vector.

The adenoviral vector desirably is administered in a composition, preferably a physiologically acceptable (e.g., pharmaceutically acceptable) composition, which comprises a carrier, preferably a physiologically (e.g., pharmaceutically) acceptable carrier and the adenoviral vector(s). Any suitable carrier can be used within the context of the invention, and such carriers are well known in the art. The choice of carrier will be determined, in part, by the particular site to which the composition is to be administered and the particular method used to administer the composition. Ideally, in the context of adenoviral vectors, the composition preferably is free of replication-competent adenovirus.

Suitable formulations for the composition include aqueous and non-aqueous solutions, isotonic sterile solutions, which can contain anti-oxidants, buffers, and bacteriostats, and aqueous and non-aqueous sterile suspensions that can include suspending agents, solubilizers, thickening agents, stabilizers, and preservatives. The formulations can be presented in unit-dose or multi-dose sealed containers, such as ampules and vials, and can be stored in a freeze-dried (lyophilized) condition requiring only the addition of the sterile liquid carrier, for example, water, immediately prior to use. Extemporaneous solutions and suspensions can be prepared from sterile powders, granules, and tablets of the kind previously described. Preferably, the carrier is a buffered saline solution. More preferably, the expression vector for use in the inventive method is administered in a composition formulated to protect the expression vector from damage prior to administration. For example, the composition can be formulated to reduce loss of the adenoviral vector on devices used to prepare, store, or administer the expression vector, such as glassware, syringes, or needles. The composition can be formulated to decrease the light sensitivity and/or temperature sensitivity of the expression vector. To this end, the composition preferably comprises a pharmaceutically acceptable liquid carrier, such as, for example, those described above, and a stabilizing agent selected from the group consisting of polysorbate 80, L-arginine, polyvinylpyrrolidone, trehalose, and combinations thereof. Use of such a composition will extend the shelf life of the vector, facilitate administration, and increase the efficiency of the inventive method. Formulations for adenoviral vector-containing compositions are further described in, for example, U.S. Patent 6,225,289, 6,514,943, U.S. Patent Application Publication No. 2003/4153065 A1, and International Patent Application No. WO 00/34444. A composition also can be formulated to enhance transduction efficiency. In addition, one of ordinary skill in the art will appreciate that the adenoviral vector can be present in a composition with other therapeutic or biologically-active agents. For example, factors that control inflammation, such as ibuprofen or steroids, can be part of the composition to reduce swelling and inflammation associated with *in vivo* administration of the viral vector. Immune system stimulators can be administered to enhance any immune response to the antigen. Antibiotics, i.e., microbicides and fungicides, can be present to treat existing infection and/or reduce the risk of future infection, such as infection associated with gene transfer procedures.

The construction of adenoviral vectors is well understood in the art. Adenoviral vectors can be constructed and/or purified using the methods set forth, for example, in U.S. Patents 5,965,358, 6,168,941, 6,329,200, 6,383,795, 6,440,728, 6,447,995, and 6,475,757, and U.S. Patent Application Publication Nos. 2003/0203469 A1 and 2003/0203480 A1, and International Patent Applications WO 98/53087, WO 98/56937, WO 99/15686, WO 99/54441, WO 00/12765, WO 01/77304, and WO 02/29388, as well as the other references identified herein. A replication-deficient adenoviral vector can be propagated in a complementing cell line which complements for the gene functions for which the adenoviral vector is deficient. Desirably, the complementing cell line comprises, integrated into the cellular genome, adenoviral nucleic acid sequences which encode gene functions required for adenoviral propagation. The source of the adenoviral nucleic acid sequences in the complementing cell line can be an adenovirus of the same subgroup and/or serotype of the adenoviral vector. For example, an E1-deficient, serotype 35 adenoviral vector can be propagated in a complementing cell line comprising the E1 coding sequences of a serotype 35 adenovirus. Preferably, the complementing cell line, which allows for the propagation of a replication-deficient adenoviral vector of the invention, specifically complements for those functions that are missing from the replication-deficient adenoviral vector of interest. Such a cell line also preferably contains the complementing gene(s) in a nonoverlapping fashion so as to minimize, if not eliminate, the possibility of vector recombination yielding a replication-competent adenoviral vector, as disclosed in U.S. Patent 5,994,106. Yet, the cellular genome need not comprise nucleic acid sequences, the gene products of which complement for all of the deficiencies of a replication-deficient adenoviral vector. One or more replication-essential gene functions lacking in a replication-deficient adenoviral vector can be supplied by a helper virus, e.g., an adenoviral vector that supplies in *trans* one or more essential gene functions required for replication of the desired adenoviral vector. Helper virus is often engineered to prevent packaging of infectious helper virus. For example, one or more replication-essential gene functions of the E1 region of the adenoviral genome are provided by the cell, while one or more replication-essential gene functions if the E4 region of the adenoviral genome are provided by a helper virus.

It also is possible to propagate non-subgroup C adenoviral vectors in complementing cell lines designed for replication-deficient group C adenoviral vectors. Indeed, it has been discovered that replication-deficient non-subgroup C adenoviral vectors can be propagated in complementing cell lines generated using subgroup C adenovirus coding sequences. This discovery was unexpected in view of the substantial differences between non-group C adenoviruses and group C adenoviruses, as exemplified by the relatively small amount of similarity between the E1A and E1B gene products of non-group C adenoviruses and group C adenoviruses.

In this regard, the invention provides a method of producing an adenoviral vector. The method comprises introducing an adenoviral vector (e.g., a serotype 41 adenoviral vector or a serotype 35 adenoviral vector) into a cell. The adenoviral vector comprises an adenoviral genome deficient in one or more replication-essential gene functions of the E1A region of the adenoviral genome and one or more replication-essential gene functions of the E1B region of the adenoviral genome encoding the E1B 55K protein. The adenoviral vector can comprise further deficiencies as described herein (e.g., deficiencies in one or more replication-essential gene function of the E4 region). Preferably, the adenoviral vector is deficient in all essential gene functions of the E1A and E1B regions of the adenoviral genome. E1A and E1B region coding sequences of serotype 35 and serotype 41 adenovirus are known in the art and can be removed or mutated to interrupt functioning of the encoded gene products. For example, the E1B 55K coding sequence of serotype 35 adenovirus is located at approximately nucleotides 1916-3400 of the serotype 35 adenoviral genome. The serotype 41 adenoviral genome sequence is available in GenBank as Accession No. M18289. The nucleic acid sequence encoding the E1B 55K protein is located at approximately nucleotides 1731-3149 of the serotype 41 adenoviral genome.

The cell of the inventive method comprises a subgroup C adenoviral nucleic acid sequence encoding the one or more essential gene functions of the E1 region which are deficient in the adenoviral vector (e.g., the cell produces essential subgroup C adenoviral E1A gene products and at least the 55K protein encoded by the E1B that are not produced by the adenoviral vector), as well as a subgroup C adenoviral nucleic acid sequence encoding essential gene functions of other regions which are deficient in the adenoviral vector. The cell further comprises open reading frame 6 (ORF6) of a subgroup C adenoviral E4 region. An exemplary cell line appropriate for use in the inventive method is a 293 cell line comprising adenoviral E4 ORF6, such as the 293-ORF6 cell line described in Brough et al., Journal of Virology, 70(9), 6497-6501 (1996). Alternatively or in addition, the cell comprises ORF3 of a subgroup C adenoviral E4 region. It will be appreciated that the essential gene functions need not be encoded by a single nucleic acid sequence, but can be encoded by multiple nucleic acid sequences which work in concert to supply the required replication-essential gene functions. Likewise, one or more replication-essential gene functions can be provided in *trans* by another gene transfer vector, e.g., a plasmid or helper virus. The cell of the inventive method does not comprise non-subgroup C nucleic acid sequences encoding any or all of the essential gene functions deficient in the adenoviral vector. The method further comprises propagating the adenoviral vector, which can be isolated and formulated in a composition for administration to a mammal.

While the method of producing an adenoviral vector is described herein with respect to serotype 35 adenoviral vectors and serotype 41 adenoviral vectors, a cell producing subgroup C (preferably serotype 5) E1A, E1B, and E4 ORF6 (and/or ORF3) gene products can support replication of adenoviral vectors of any subgroup (subgroup A, subgroup B, subgroup C, subgroup D, subgroup E, and subgroup F) which comprise deficiencies in the E1A and, optionally, E1B regions of the adenoviral genome. Use of a system comprising non-group C adenoviral vectors and cells comprising subgroup C adenoviral nucleic acid sequences diminishes the likelihood of producing stocks of adenoviral vector comprising replication competent adenovirus (RCA) contamination.

The invention also provides a composition, preferably a pharmaceutical composition, comprising a serotype 41 or serotype 35 adenoviral vector comprising an adenoviral genome deficient in one or more replication-essential gene functions of the E1A region of the adenoviral genome and the E1B region of the adenoviral genome encoding the E1B 55K protein. The composition further comprises a carrier (e.g., a pharmaceutically or physiologically acceptable carrier). Ideally, the composition comprises at least about 1x10⁵ intact serotype 41 or serotype 35 adenoviral vector particles (e.g., at least about 1x10⁵-1x10¹² serotype 41 or serotype 35 adenoviral vector particles), at least about 1x10⁶ serotype 41 or serotype 35 adenoviral vector particles (e.g., at least about 1x10⁶-1x10¹¹ serotype 41 or serotype 35 adenoviral vector particles, preferably at least about 1x10⁷ serotype 41 or serotype 35 adenoviral vector particles), or at least about 1x10⁸ serotype 41 or serotype 35 adenoviral vector particles (e.g., at least about 1x10⁸-1x10¹⁰ serotype 41 or serotype 35 adenoviral vector particles, preferably about 1x10⁹ serotype 41 or serotype 35 adenoviral vector particles). The replication-deficient serotype 41 or serotype 35 adenoviral vector can comprise any of the characteristics described above with respect to adenoviral vectors, including deficiencies in replication-essential functions encoded by other regions of the adenoviral genome. Methods of generating non-group C adenoviral vectors is further described in U.S. Patents 5,837,511 and 5,849,561, and International Patent Applications WO 97/12986 and WO 98/53087.

The following examples further illustrate the invention but, of course, should not be construed as in any way limiting its scope

### EXAMPLE 1

This example demonstrates the generation of an E1-deleted adenoviral vector based on a subgroup F adenovirus.

The full genome of a desired serotype 41 (Ad41) adenoviral vector was constructed as a single plasmid. First, the full-length Ad41 wild-type genome was constructed in plasmid form. The wild-type Ad41 genome was recombined into a small plasmid by the AdRescue method. Briefly, the small plasmid (i.e., the "recipient plasmid"), pAd41RSQ.BN, was constructed through several sub-cloning steps. Ad41 genome sequences corresponding to the first 405 base pairs (bp) of the wild-type Ad41 genome were amplified by polymerase chain reaction (PCR) and TOPO-cloned (Invitrogen) into pCR2.1TOPO, thereby creating pCR2.1TOPO.Ad41Left. The left end of the Ad41 adenoviral genome was subcloned by XhoI restriction digestion cloning into pKSII to create plasmid pKSIIAd41(1-405)Left. The EcoRI/PmeI fragment from pKSIIAd35(1-446)Left.Cos (see Example 2) containing a lambda cos site and lac Iq was subcloned into EcoRI/PmeI digested pKSIIAd41(1-405)Left to create the plasmid pKSIIAd41(1-405)Left.IqCos. Ad41 genome sequences corresponding to the right terminal 440 bp were PCR amplified, TOPO-cloned into pCR2.1TOPO and subcloned by XbaI restriction digestion into pKSIIAd41(1-405)Left.IqCos to create the plasmid pKSIIAd41(1-405)Left.IqCos.Ad41(440bp)Right. A positive/negative selection cassette, BN (described in McVey, et. al., Journal of Virology, 76,3670-3677 (2002)), was then cloned between the Ad41 Left end genomic sequences and Right end genomic sequences at the SalI site to generate pKSAd41RSQ.BN. Finally, the pBluescript plasmid backbone was replaced with a p15A DNA replication origin and kanamycin resistance plasmid backbone to generate pAd41RSQ.BN. The wild-type full length genome of Ad41 was rescued into plasmid form by homologous recombination with pAd4IRSQ.BN. Rec+ *E. coli* were co-transformed with Ad41 wild-type DNA and SwaI-linearized pAd4IRSQ.BN. Recombinant plasmids with the full-length Ad41 genome replacing the BN selection cassette were identified by drug selection of bacteria and restriction enzyme analysis of miniprep plasmid DNA. Two plasmids were identified, pAC41-16 and pAC41-13B. 293-ORF6 cells were transfected with these plasmid clones. Cytopathic effect (cpe), indicative of viral propagation, was observed by 6 days post-transfection. Passaging of the transfection lysate resulted in cpe of fresh 293-ORF6 cells in a dose-dependent manner. PCR analysis verified the presence of Ad41 and the absence of serotype 5 (Ad5) adenoviral vector nucleotide sequences in the resulting adenoviral vectors. Transfection of 293 cells with pAC41-16 and pAC41-13B did not result in cpe.

To construct the full genome of an E1-deleted Ad41 adenovector that expressed the green fluorescent protein (GFP), pAC41-13B was recombined with Ad41 shuttle plasmids, which contain the left-most 5058 bp of Ad41 and the BN selection cassette inserted at the deletion junction of nucleotides 361-3164. The resultant base plasmid, pAC41-13BE1 (BNot), was recombined in *E. coli* with an Ad41 shuttle plasmid, specifically pAd41E1(GFP)dpme containing, in place of the BN expression cassette, a CMV enhancer-promoter-GFP-bovine growth hormone (BGH) polyA expression cassette. The resultant Ad41 E1-deleted (lacking nucleotides 360 through 3165) adenovector genome plasmid, pAC41-13BE1(f), was digested with PmeI and transfected into 293-ORF6 cells (described in, for example, International Patent Application WO 95/34671 and Brough et al., Journal of Virology, 70(9), 6497-6501 (1996). Clusters of GFP positive cells were evident by 5 days post-transfection. Passaging the transfected cell lysate onto fresh 293-ORF6 cells resulted in the appearance of many clusters of GFP positive cells, consistent in appearance with early adenovirus plaque formation. Subsequent passaging of the infection lysate to fresh 293-ORF6 cells resulted in a large number of individual GFP positive cells 24 hours post-infection and many clusters of GFP positive cells by 5 days post-infection. The presence of the Ad41 adenoviral vector was confirmed by PCR, and the absence of Ad5 nucleic acid sequences was confirmed by PCR.

This example illustrates a method of constructing a subgroup F, serotype 41, adenoviral vector deficient in all essential gene functions of the E1 region of the adenoviral genome. The adenoviral vector was propagated in 293-ORF6 cells to create a stock of Ad41 adenoviral vectors.

### EXAMPLE 2

This example demonstrates the generation of an E1-deleted adenoviral vector based on a subgroup B adenovirus.

The full genome of a desired serotype 35 (Ad35) adenoviral vector was constructed as a single plasmid. First, the full-length Ad35 wild-type adenoviral genome was constructed in plasmid form. The wild-type Ad35 adenoviral genome was recombined into a small plasmid by the AdRescue method. Briefly, the small plasmid (i.e., the "recipient plasmid"), pAd35RSQ.BN, was constructed through several sub-cloning steps. Ad35 genomic sequences corresponding to the first 446 bp of the wild-type Ad35 adenoviral genome were amplified by PCR and TOPO-cloned (Invitrogen) into pCR2.1TOPO to create pCR2.1TOPO.Ad35Left. The Ad35 left end genomic sequence was subcloned by XhoI restriction digestion cloning into pKSII to create plasmid pKSIIAd35(1-446)Left. The AscI fragment from GenVec's pACE series of plasmids (McVey et al., Journal of Virology, 76(8), 3670 (2002)) containing a lambda cos site and lac Iq was subcloned into AscI/PacI digested pKSIIAd35(1-446)Left to make the plasmid pKSIIAd35(1-446)Left.IqCos. Ad35 genomic sequences corresponding to the right end terminal 440 bp were PCR amplified and subcloned by XbaI restriction digestion into pKSIIAd35(1-446)Left.IqCos to create the plasmid pKSIIAd35(1-446)Left.IqCos.Ad35(440bp)Right. The BN selection cassette was then cloned between the Ad35 Left and Right genome sequences at the SalI site to generate pKSAd35RSQ.BN. Finally, the pBluescript plasmid backbone was replaced with a p15A DNA replication origin and kanamycin resistance plasmid backbone to make pAd35RSQ.BN. The wild-type full length Ad35 genome was rescued into plasmid form by homologous recombination with pAd35RSQ.BN. Rec+ *E. coli* were co-transformed with Ad35 wild-type DNA and SwaI-linearized pAd35RSQ.BN. Recombinant plasmids with the full-length Ad35 genome replacing the BN selection cassette were identified by drug selection of bacteria and restriction enzyme analysis of miniprep plasmid DNA. Such a plasmid was identified as pAC35-6. 293-ORF6 cells were transfected with this plasmid, and cytopathic effect (cpe) was observed by 2 days post-transfection. Passaging of the transfection lysate resulted in cpe of fresh 293-ORF6 cells in a dose-dependent manner. PCR analysis verified the presence of the Ad35 adenoviral vector and the absence of Ad5 DNA.

A plasmid containing the full genome of an E1-deleted Ad35 adenoviral vector that expressed the green fluorescent protein (GFP), pAC35-6E1(f) was constructed by similar methods as described in Example 1. The plasmid, containing a deletion of Ad35 nucleotide sequences 447 through 3417, was digested with PmeI and transfected into 293-ORF6 cells. Clusters of GFP positive cells were evident by 3 days post-transfection. Passaging the transfected cell lysate onto 1 x 10⁶ fresh 293-ORF6 cells resulted in the transduction of the cells by GFP and the appearance of cytopathosis in a dose dependent manner. Infection with 50 µl of transfection lysate resulted in nearly 100% of cells being GFP positive and approximately 70% of the cells undergoing a cytopathic effect at about 24 hours post-infection. This is consistent with the transfection lysate having an infectious titer of at least 2 x 10⁷ GFP-transducing units per ml, corresponding to a multiplicity of infection of 1 GFP-transducing unit per cell. Infection with 450 µl resulted in 100% of cells being GFP positive and about 95% of cells undergoing a cytopathic effect by 24 hours post-infection. The presence of the Ad35 adenoviral vector was confirmed by PCR, and the absence of Ad5 DNA was confirmed by PCR.

This example illustrates a method of constructing a subgroup B, serotype 35, adenoviral vector deficient in all essential gene functions of the E1 region of the adenoviral genome. The adenoviral vector was propagated in 293-ORF6 cells to create a stock of Ad35 adenoviral vectors.

### EXAMPLE 3

This example demonstrates the generation of an E1/E3-deleted adenoviral vector based on a subgroup B adenovirus.

Ad35 (GenBank Accession #AY128640) has five XbaI sites at nucleotides 23698, 27240, 27924, 28735 and 29726. By partial XbaI digestion of Ad35, ligation and in vitro packaging of pAC35E1 ("BN cassette," discussed above), an E3 (XbaI) deletion was generated in the Ad35 virus that spans 2486 bp of sequences contained between nucleotides 27241 and 29726. This E3 XbaI deletion removes nucleic acid sequences that encode the E3 15K, 18.5K, and 20.3K proteins. The BN cassette in the E1 region was replaced with an expression cassette containing a CMV promoter driving expression of HIV gp140dCFIdv12(B) to create pAC35E1(RL.CMVint.gp140B.SV40)E3(Xba). 293-ORF6 cells were transfected with this plasmid and a cytopathic effect was observed on the first passage following transfection. The Ad35gp140(B).E3(Xba) vector was expanded over one additional passage and the presence of the vector was confirmed by PCR and western blot analysis of gp140(B) expression. The deletion of the E1 and E3 regions was verified by PCR analyses that were specific for both the transgene and for the deletions. The cell-virus lysate from the second passage post-transfection was used to inoculate a fresh plate of 293 cells. Protein extracts were prepared from the infected cells at 24 hours post-infection, electrophoresed through a 7.5% SDS-PAGE gel, blotted to PVDF membrane, and probed with an anti-HIV antibody. The gp140 protein was detected in the protein extracts.

This example illustrates a method of constructing a subgroup B adenoviral vector deficient in all essential gene functions of the E1 region of the adenoviral genome and deficient in the E3 region of the adenoviral genome.

### EXAMPLE 4

This example demonstrates the generation of an E1/E4-deleted adenoviral vector based on a subgroup B adenovirus.

Two deletions of the E4 region of the Ad35 adenoviral genome were generated, each in combination with a deletion of the E1 region of the Ad35 adenoviral genome. In this respect, an expression cassette comprising a CMV promoter controlling expression of a nucleic acid sequence encoding either gp140dCFIdV12(B), luciferase, secreted alkaline phosphatase, beta-galactosidase, or beta-glucuronidase was inserted within an E1 region deletion of the Ad35 genome as described herein. The first E4 deletion, Ad35 E4(dOrf26) was constructed by generating a 1749 bp deletion from a NruI restriction endonuclease site at nucleotide 32011 (see GenBank Accession #AY128640), which is located in the non-coding sequences between the fiber gene and the E4 region, to a SmaI site at nucleotide 33760 (see GenBank Accession #AY128640), which is located within the coding sequences for the Ad35 E4 ORF2 protein. The Ad35 ORF2 protein does not share homologies to the Ad5 E4 ORF2 protein. The second E4 deletion, Ad35 E4(dOrf1-6), was constructed by generating a targeted deletion of 2408 bp, which removed all E4 coding sequences from the NruI site described above to the first adenine (A) at nucleotide 34419 (see GenBank Accession #AY128640) in the ATG codon of the Ad35 E4 ORF1.

The Ad35 vector genomic plasmids containing the E1 and E4 deletions were transfected into 293-ORF6 cells. Subsequent serial passaging of the transfection lysates demonstrated that the E1/E4-deleted adenoviral vectors were viable regardless of the E4 deletion and the transgene expressed from the E1 region. By one or two passages post-transfection, 100% of the cells underwent a cytopathic effect, induced by the growth and spread of the E1/E4-deleted Ad35 vectors. The presence of each vector was determined by PCR analysis. Deletion of the E1 and E4 regions in each vector was verified by PCR analyses that also positively identified the presence of the transgene.

This example illustrates a method of constructing a subgroup B adenoviral vector deficient in all essential gene functions of the E1 region and the E4 region of the adenoviral genome.

### EXAMPLE 5

This example demonstrates the generation of an E1-deleted adenoviral vector based on a subgroup B adenovirus that comprises a nucleic acid sequence encoding a pIX protein operably linked to a heterologous promoter.

E1-deleted Ad35 vectors encoding β-galactosidase (LacZ), β-glucuronidase (GUS), or the HIV envelope protein gp140dCFIdV12(B) (gp 140B) each operably linked to a CMV promoter were constructed as described above and were propagated in 293-ORF6 cells. Successful rescue of Ad35 vectors is defined as the induction of cytopathic effects (cpe) within 48 hours of the first passage after initial transfection of plasmid DNA in 293-ORF6 cells. The Ad35 vectors encoding LacZ, GUS, or HIV gp140B were not rescued. The LacZ, GUS, and gp140B genes were successfully rescued into E1-deleted and E1/E4-deleted Ad5 vectors.

To determine the stability of the Ad35 vectors encoding LacZ, GUS, and gp140B, a thermostability assay was performed. In this regard, E1-deleted Ad35 vectors were propagated in 293-ORF6 cells and purified via isopycnic gradient centrifugation (three sequential cesium chloride gradients), dialysed against four exchanges of Final Formulation Buffer (FFB), and stored at -80° C. The vectors were thawed on ice and diluted to approximately 1x10¹¹ particle units (pu)/ml (a minimum dilution of 1:10) with ice-cold phosphate buffered saline (PBS) to dilute the stabilizing agent in the FFB. Samples of diluted vector were placed at 48° C, removed from the water bath, and stabilized by equal volume dilution with PBS and 10% fetal bovine serum. The samples were assayed for infectivity in a fluorescent focus forming assay by indirect immunofluorescent detection of the hexon protein in infected cells.

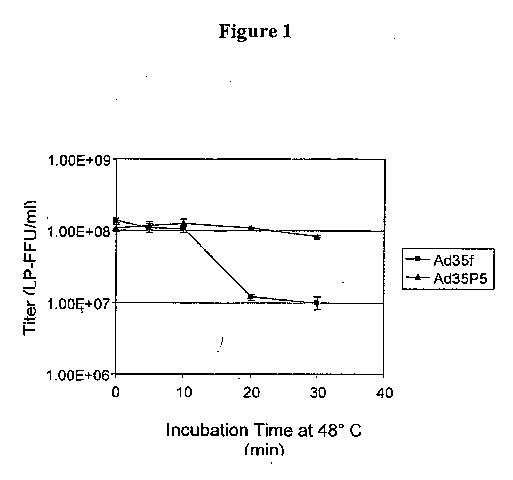

The results of this analysis demonstrated that the E1-deleted Ad35 vectors were thermolabile (see Figure 1). To confirm the thermolabile phenotype observation, a purified preparation of a thermolabile vector was analyzed by reverse-phase chromatography and mass spectroscopy (MS). In this respect, approximately 1x10¹¹ particles of wild-type adenovirus type 35 and E1-deleted Ad35 vector were fractioned over a C4 column (Phenomenex, Torrance, CA) and collected for subsequent MS analysis. After the fractions were lyophilized, the samples were reconstituted in 0.1 % trifluoroacetic acid, 50% acetonitrile, and 1% sinapinic acid (MALDI matrix). One microliter of reconstituted sample plus matrix were placed on a MALDI target plate and allowed to dry. Mass spectroscopy (MS) analysis was performed using a Voyager DE-Pro MALDI MS spectrometer (Applied Biosystems, Foster City, CA). The abundance of protein IX was normalized to hexon abundance (the fractions were concomitant). Two peaks where observed (see Figure 2): a peak at 14.1kDa/z corresponding to the singly charged protein IX, and a peak at 7 kDa/z corresponding to the doubly charged protein IX. Protein IX was significantly less abundant in the E1-deleted Ad35 vector than the amount in the Ad35 wild-type virus control (see Figure 2).

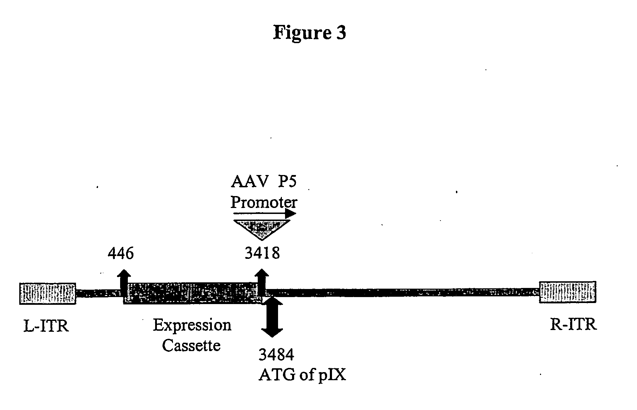

The adeno-associated virus (AAV) p5 promoter (SEQ ID NO: 11) was introduced into the E1-deleted Ad35 vectors 3' of the CMV expression cassette (see Figure 3), which produced a thermostable phenotype (see Figure 1). In addition, introduction of the p5 promoter resulted in the successful rescue of the E1-deleted Ad35 vector that expressed the HIV gene gp140(B). High viral yields of up to 40,000 particles/cell following purification through three cesium chloride gradients were obtained.

This example demonstrates the thermostability of an E1-deleted adenoviral vector based on a subgroup B adenovirus that comprises a nucleic acid sequence encoding pIX protein operably linked to a heterologous promoter.

### EXAMPLE 6

This example demonstrates the enhanced packaging capacity of an E1-deleted Ad35 adenoviral vector that comprises a nucleic acid sequence encoding a pIX protein operably linked to a heterologous promoter.

Several Ad35 vectors with genome sizes of 99.8% or below the genome size of wild-type Ad35 met the criterion for successful rescue and propagation as described in Example 5. These vectors included (a) E1-deleted vectors, (b) E1-deleted adenoviral vectors containing an expression cassette positioned in the deleted E1 region in either a 5'-3' or 3'-5' orientation with respect to the direction of E1 transcription, (c) adenoviral vectors comprising the RGD ligand conjugated to the HI loop or to the C-terminus of the fiber protein, and (d) adenoviral vectors comprising a chimeric Ad35/Ad5 fiber protein. Ad35 vectors with larger genome sizes were not successfully rescued, as demonstrated by genetic rearrangement of the genomes following transfection and passaging. The results of this analysis are set forth below in Table 1.

**Table 1**

| Vector | Genome size | % of wild-type (34794 nucleotides) | Genetic Stability |
|---|---|---|---|
| Ad35.null | 33,025 | 94.92% | Yes |
| Ad35(3326)F(5K) | 33,594 | 96.55% | Yes |
| Ad35.f | 33,736 | 96.96% | Yes |
| Ad35.(3326f).F(HI-GRGD) | 33,745 | 96.99% | Yes |
| Ad35.(3326).F(C-RGD) | 33,757 | 97.02% | Yes |
| Ad35.f3326 | 33,828 | 97.22% | Yes |
| Ad35(3326f)F(5S.5K) | 34,392 | 98.84% | Yes |
| Ad35(3326f)F(5S) | 34,419 | 98.92% | Yes |
| Ad35.S | 34,561 | 99.33% | Yes |
| Ad35(3418gp140B.ori2)F(5K) | 34,581 | 99.39% | Yes |
| Ad35(3326gp1408.ori2)F(5K) | 34,668 | 99.64% | Yes |
| Ad35.L | 34,721 | 99.79% | Yes |
| Ad35.G | 34,774 | 99.94% | No |
| Ad35.gp140B | 34,776 | 99.95% | No |
| Ad35.gp140B.F(HI-RGD) | 34,799 | 100.01% | No |
| Ad35.gp140B.F(HI3G4CRGD) | 34,856 | 100.18% | No |
| Ad35.gp140B.F(C5GS4CRGD) | 34,868 | 100.21% | No |
| Ad35.gp140A | 34,938 | 100.41% | No |
| Ad35.Z | 36,075 | 103.68% | No |

Regardless of genome size, the E1-deleted Ad35 vectors were thermolabile (see Figure 1). To confirm the thermolabile phenotype observation, a purified preparation of an El-deleted small-genome vector, Ad35.f, was analyzed by SDS-PAGE analysis. Specifically, 2.5x10¹⁰ particles of wild-type Ad35 and Ad35.fwere loaded onto a 4-12% gradient acrylamide gel under denaturing conditions (samples were also pretreated with SDS and DTT at 100° C for 10 min). After chromatography at 180V for approximately one hour, the gel was stained with the PlusOne™ silver staining kit according to the manufacturer's (Amersham Biosciences, Piscataway, NJ) instructions. The Ad35.f vector also was analyzed by reverse-phase chromatography with mass spectroscopy as described in Example 5. Protein IX was detected in this vector but was significantly less abundant than the amount in the Ad35 wild-type virus control (see Figures 2 and 4).

To improve thermostability, the adeno-associated virus (AAV) p5 promoter (SEQ ID NO: 11) was introduced into an E1-deleted Ad35 vector 3' of the expression cassette (see Figure 3). The resultant vector, Ad35P5, comprised a genome size that was 100.43% of the wild-type Ad35 genome, and was tested for thermostability as described in Example 5. The Ad35P5 vector exhibited thermostability (see Figure 1), and was efficiently rescued. High viral yields of up to 40,000 particles/cell following purification through three cesium chloride gradients were obtained.

This example demonstrates the increased packaging capacity and thermostability of an E1-deleted Ad35 adenoviral vector that comprises a nucleic acid sequence encoding a pIX protein operably linked to a heterologous promoter.

### EXAMPLE 7

This example describes the effect of E1 deletions proximal to the pIX gene on viral stability in an Ad41 adenoviral vector.

E1-deleted Ad41 adenoviral vectors containing an expression cassette comprising the gene encoding green fluorescent protein (GFP) under the control of a CMV enhancer/promoter were constructed by homologous recombination in *E. coli* using methods known in the art. Four different E1 deletions were generated, and the CMV-GFP cassette was positioned in the deleted E1 region in either a left to right or right to left orientation. The adenoviral vectors were tested for rescue, propagation, and stability as described herein. The results of this analysis are set forth in Table 2.

The deletion junction on the left side of the E1 region did not have an effect on the rescue, expansion, or genetic stability of the Ad41 vector genome. In contrast, the deletion junction on the right side of the E1 region affected the rescue and genetic stability of the vectors. Namely, the Ad41 vectors deleted at nucleotide 3100 were efficiently rescued as evidenced by the appearance of cytopathic effect on the first or second passage post-transfection of the genomic plasmid into 293-ORF6 cells (see Table 2, lines 1 and 5). Similarly, the Ad41 genomes with the nucleotide 3100 deletion junction were genetically stable, as assessed by PCR analysis of the expression cassette region, through the second passage post-transfection (see Table 2, lines 1 and 5) and through a third passage post-transfection (data not shown). In contrast, Ad41 vectors with a right side deletion junction at nucleotide 3165 (i.e., 65 nucleotides closer to the start codon of protein IX) were not viable or genetically stable. The Ad41 vectors comprising the CMV expression cassette in the right-to-left orientation (i.e., the CMV enhancer abutting the protein IX gene region) were efficiently rescued, but these vectors were genetically unstable in the deleted E1 region containing the expression cassette (see Table 2, lines 2 and 3). An Ad41 vector containing the same E1 deletion but having the CMV expression cassette in the left-to-right orientation (i.e., CMV enhancer abutting the E1A residual enhancer) could not be rescued (see Table 2, line 4). Thus, the nucleotide 3100 deletion junction did not significantly affect the function of the Ad41 vector. The nucleotide 3165 deletion junction affected the viability of the vector genome, which could be partially compensated for by the presence of the bidirectional CMV enhancer proximal to the protein IX gene.

**Table 2.**

| | Coordinates | | | | | | | | | | |
|---|---|---|---|---|---|---|---|---|---|---|---|
| | Left | | Right | | Orientation of CMV cassette | | cpe | | PCR (P2) | | N |
| 1 | 480 | | 3100 | | Right to left | | + | | + | | 5 |
| 2 | 480 | | 3165 | | Right to left | | + | | Δ | | 2 |
| 3 | 360 | | 3165 | | Right to left | | + | | Δ | | 2 |
| 4 | 360 | | 3165 | | Left to right | | - | | n.a. | | 1 |
| 5 | 360 | | 3100 | | Right to left | | + | | + | | 5 |
| N = number of Ad41 vectors attempted; Δ = deletion; n.a. = not applicable | | | | | | | | | | | |

This example demonstrates that a subgroup F adenoviral vector containing a deletion proximal to the nucleic acid sequence encoding pIX has reduced viability and genetic stability.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate.

## Claims

1. Use of a non-subgroup C adenoviral vector in the manufacturing of a pharmaceutical composition for inducing an immune response in a mammal, wherein (a) the non-subgroup C adenoviral vector comprises an adenoviral fiber protein comprising an amino acid sequence comprising 80% or more identity to an amino acid sequence of a subgroup C adenoviral fiber protein, (b) the adenoviral fiber protein binds to a coxsackievirus and adenovirus receptor (CAR), and (c) the adenoviral vector further comprises a nucleic acid sequence encoding an antigen which is to be expressed in the mammal to induce an immune response.

2. The use of claim 1, wherein the adenoviral vector is a subgroup B adenoviral vector or a subgroup F adenoviral vector.

3. The use of claim 2, wherein the adenoviral vector is a serotype 35 adenoviral vector.

4. The use of any of claims 1-3, wherein the subgroup C adenoviral fiber protein is a serotype 5 adenoviral fiber protein.

5. The use of claim 4, wherein the adenoviral fiber protein comprises a shaft domain of a serotype 5 fiber protein and a knob domain of a serotype 5 fiber protein.

6. The use of claim 5, wherein the adenoviral fiber protein comprises a shaft domain of a serotype 5 fiber protein, a tail domain of a serotype 35 fiber protein, and a knob domain of a serotype 5 fiber protein.

7. The use of claim 2, wherein the adenoviral vector is a serotype 41 adenoviral vector.

8. The use of any of claims 1-7, wherein the adenoviral vector comprises an adenoviral genome that is deficient in one or more replication-essential gene functions of the E1 region and/or the E4 region of the adenoviral genome.

9. The use of claim 8, wherein the nucleic acid sequence encoding the antigen is positioned in the E1 region or the E4 region of the adenoviral genome.

10. The use of any of claims 1-9, wherein a priming gene transfer vector comprising a nucleic acid sequence encoding an antigen is to be administered to the mammal prior to administering to the mammal the adenoviral vector.

11. The use of claim 10, wherein the priming gene transfer vector comprises a nucleic acid sequence encoding a first antigen, the adenoviral vector comprises a nucleic acid sequence encoding a second antigen, and the first antigen and the second antigen are the same antigen.

12. The use of claim 10, wherein the priming gene transfer vector comprises a nucleic acid sequence encoding a first antigen, the adenoviral vector comprises a nucleic acid sequence encoding a second antigen, and the first antigen and the second antigen are different.

13. The use of any of claims 10-12, wherein the priming gene transfer vector is an adenoviral vector.

14. The use of any of claims 1-13, wherein multiple adenoviral vectors, each adenoviral vector comprising one or more nucleic acid sequences encoding one or more antigens, are to be administered to the mammal.

15. The use of any of claims 1-14, wherein the adenoviral vector comprises a chimeric adenoviral coat protein comprising a non-native amino acid sequence encoding an antigen.

16. The use of claim 15, wherein at least one antigen is presented via major histocompatibility type I complexes (MHC I) and at least one antigen is presented via MHC II complexes.

17. The use of any of claims 1-16, wherein the adenoviral vector comprises an adenoviral genome lacking native nucleic acid sequences encoding adenoviral proteins.

18. The use of any of claims 8-17, wherein a spacer sequence is positioned in the E4 region of the adenoviral genome.

19. The use of any of claims 1-18, wherein the adenoviral vector comprises multiple nucleic acid sequences encoding different antigens.

20. The use of claim 19, wherein two or more nucleic acid sequences encoding different antigens are operably linked to different promoters.

21. The use of any of claims 1-18, wherein the adenoviral vector comprises multiple nucleic acid sequences encoding the same antigen.

22. The use of claim 21, wherein two or more nucleic acid sequences encoding the same antigen are operably linked to different promoters.

23. The use of any of claims 1-22, wherein the adenoviral vector comprises a chimeric adenoviral coat protein comprising a non-native amino acid sequence, and wherein the non-native amino acid sequence comprises an RGD motif.

## Patentansprüche

1. Verwendung eines adenoviralen Vektors, der nicht der Subgruppe C angehört, bei der Herstellung einer pharmazeutischen Zusammensetzung zum Induzieren einer Immunantwort in einem Säugetier, wobei (a) der nicht der Subgruppe C angehörende adenovirale Vektor ein adenovirales Fiber-Protein mit einer Aminosäuresequenz umfasst, die eine Identität von 80% oder mehr zu einer Aminosäuresequenz eines adenoviralen Fiber-Proteins der Subgruppe C aufweist, (b) das adenovirale Fiber-Protein an einen Coxsackievirus- und Adenovirus-Rezeptor (CAR) bindet, und (c) der adenovirale Vektor ferner eine Nukleinsäuresequenz umfasst, welche für ein Antigen kodiert, das in dem Säugetier zur Induzierung einer Immunantwort zu exprimieren ist.

2. Verwendung nach Anspruch 1, wobei der adenovirale Vektor ein adenoviraler Vektor der Subgruppe B oder ein adenoviraler Vektor der Subgruppe F ist.

3. Verwendung nach Anspruch 2, wobei der adenovirale Vektor ein adenoviraler Vektor vom Serotyp 35 ist.

4. Verwendung nach einem der Ansprüche 1 bis 3, wobei das adenovirale Fiber-Protein der Subgruppe C ein adenovirales Fiber-Protein vom Serotyp 5 ist.

5. Verwendung nach Anspruch 4, wobei das adenovirale Fiber-Protein eine Schaft-Domäne eines Fiber-Proteins vom Serotyp 5 und eine Kopf-Domäne eines Fiber-Proteins vom Serotyp 5 umfasst.

6. Verwendung nach Anspruch 5, wobei das adenovirale Fiber-Protein eine Schaft-Domäne eines Fiber-Proteins vom Serotyp 5, eine Schwanz-Domäne eines Fiber-Proteins vom Serotyp 35 und eine Kopf-Domäne eines Fiber-Proteins vom Serotyp 5 umfasst.

7. Verwendung nach Anspruch 2, wobei der adenovirale Vektor ein adenoviraler Vektor vom Serotyp 41 ist.

8. Verwendung nach einem der Ansprüche 1 bis 7, wobei der adenovirale Vektor ein adenovirales Genom umfasst, welches in einer oder mehreren für die Replikation essentiellen Genfunktionen der E1-Region und/oder der E4-Region des adenoviralen Genoms defizient ist.

9. Verwendung nach Anspruch 8, wobei die Nukleinsäuresequenz, die für das Antigen kodiert, in der E1-Region oder der E4-Region des adenoviralen Genoms positioniert ist.

10. Verwendung nach einem der Ansprüche 1 bis 9, wobei vor der Verabreichung des adenoviralen Vektors an das Säugetier ein Priming-Gentransfervektor an das Säugetier zu verabreichen ist, welcher eine Nukleinsäuresequenz umfasst, die für ein Antigen kodiert.

11. Verwendung nach Anspruch 10, wobei der Priming-Gentransfervektor eine Nukleinsäuresequenz umfasst, die für ein erstes Antigen kodiert, wobei der adenovirale Vektor eine Nukleinsäuresequenz umfasst, die für ein zweites Antigen codiert, und wobei das erste Antigen und das zweite Antigen dasselbe Antigen sind.

12. Verwendung nach Anspruch 10, wobei der Priming-Gentransfervektor eine Nukleinsäuresequenz umfasst, die für ein erstes Antigen kodiert, wobei der adenovirale Vektor eine Nukleinsäuresequenz umfasst,die für ein zweites Antigen kodiert, und wobei das erste Antigen und das zweite Antigen verschieden sind.

13. Verwendung nach einem der Ansprüche 10 bis 12, wobei der Priming-Gentransfervektor ein adenoviraler Vektor ist.

14. Verwendung nach einem der Ansprüche 1 bis 13, wobei mehrere adenovirale Vektoren an das Säugetier zu verabreichen sind, und wobei jeder adenovirale Vektor eine oder mehrere Nukleinsäuresequenzen umfasst, die für ein oder mehrere Antigene kodieren.

15. Verwendung nach einem der Ansprüche 1 bis 14, wobei der adenovirale Vektor ein chimäres adenovirales Hüllprotein umfasst, welches eine nicht-native Aminosäuresequenz umfasst, die für ein Antigen kodiert.

16. Verwendung nach Anspruch 15, wobei mindestens ein Antigen über Haupthistokompatibilitätskomplexe Typ I (MHC I) und mindestens ein Antigen über MHC II präsentiert wird.

17. Verwendung nach einem der Ansprüche 1 bis 16, wobei der adenovirale Vektor ein adenovirales Genom umfasst, dem native Nukleinsäuresequenzen, die für adenovirale Proteine kodieren, fehlen.

18. Verwendung nach einem der Ansprüche 8-17, wobei eine Spacer-Sequenz in der E4-Region des adenoviralen Genoms positioniert ist.

19. Verwendung nach einem der Ansprüche 1 bis 18, wobei der adenovirale Vektor mehrere Nukleinsäuresequenzen umfasst, die für verschiedene Antigene kodieren.

20. Verwendung nach Anspruch 19, wobei zwei oder mehr Nukleinsäuresequenzen, die für verschiedene Antigene kodieren, an verschiedene Promotoren funktionell gekoppelt sind.

21. Verwendung nach einem der Ansprüche 1 bis 18, wobei der adenovirale Vektor mehrere Nukleinsäuresequenzen umfasst, die für dasselbe Antigen kodieren.

22. Verwendung nach Anspruch 21, wobei zwei oder mehr Nukleinsäuresequenzen, die für dasselbe Antigen kodieren, an verschiedene Promotoren funktionell gekoppelt sind.

23. Verwendung nach einem der Ansprüche 1 bis 22, wobei der adenovirale Vektor ein chimäres adenovirales Hüllprotein umfasst, welches eine nicht-native Aminosäuresequenz umfasst, und wobei die nicht-native Aminosäuresequenz ein RGD-Motiv umfasst.

## Revendications

1. Utilisation d'un vecteur adénoviral n'appartenant pas au sous-groupe C dans la fabrication d'une composition pharmaceutique destinée à induire une réponse immunitaire chez un mammifère, dans laquelle (a) le vecteur adénoviral n'appartenant pas au sous-groupe C comprend une protéine fibre adénovirale comprenant une séquence d'acides aminés présentant 80 % d'identité ou davantage avec une séquence d'acides aminés d'une protéine fibre adénovirale du sous-groupe C, (b) la protéine fibre adénovirale se lie à un récepteur des coxsackievirus et adénovirus (CAR), et (c) le vecteur adénoviral comprend en outre une séquence d'acide nucléique codant pour un antigène qui doit être exprimé chez le mammifère afin d'induire une réponse immunitaire.

2. Utilisation selon la revendication 1, dans laquelle le vecteur adénoviral est un vecteur adénoviral du sous-groupe B ou un vecteur adénoviral du sous-groupe F.

3. Utilisation selon la revendication 2, dans laquelle le vecteur adénoviral est un vecteur adénoviral de sérotype 35.

4. Utilisation selon l'une quelconque des revendications 1-3, dans laquelle la protéine fibre adénovirale du sous-groupe C est une protéine fibre adénovirale de sérotype 5.

5. Utilisation selon la revendication 4, dans laquelle la protéine fibre adénovirale comprend un domaine de tige d'une protéine fibre de sérotype 5 et un domaine globulaire d'une protéine fibre de sérotype 5.

6. Utilisation selon la revendication 5, dans laquelle la protéine fibre adénovirale comprend un domaine de tige d'une protéine fibre de sérotype 5, un domaine de queue d'une protéine fibre de sérotype 35, et un domaine globulaire d'une protéine fibre de sérotype 5.

7. Utilisation selon la revendication 2, dans laquelle le vecteur adénoviral est un vecteur adénoviral de sérotype 41.

8. Utilisation selon l'une quelconque des revendications 1-7, dans laquelle le vecteur adénoviral comprend un génome adénoviral qui est déficient en une ou plusieurs fonctions géniques essentielles à la réplication de la région E1 et/ou la région E4 du génome adénoviral.

9. Utilisation selon la revendication 8, dans laquelle la séquence d'acide nucléique codant pour l'antigène est positionnée dans la région E1 ou la région E4 du génome adénoviral.

10. Utilisation selon l'une quelconque des revendications 1-9, dans laquelle un vecteur de transfert génique de sensibilisation comprenant une séquence d'acide nucléique codant pour un antigène doit être administré au mammifère avant d'administrer le vecteur adénoviral au mammifère.

11. Utilisation selon la revendication 10, dans laquelle le vecteur de transfert génique de sensibilisation comprend une séquence d'acide nucléique codant pour un premier antigène, le vecteur adénoviral comprend une séquence d'acide nucléique codant pour un second antigène, et le premier antigène et le second antigène sont le même antigène.

12. Utilisation selon la revendication 10, dans laquelle le vecteur de transfert génique de sensibilisation comprend une séquence d'acide nucléique codant pour un premier antigène, le vecteur adénoviral comprend une séquence d'acide nucléique codant pour un second antigène, et le premier antigène et le second antigène sont différents.

13. Utilisation selon l'une quelconque des revendications 10-12, dans laquelle le vecteur de transfert génique de sensibilisation est un vecteur adénoviral.

14. Utilisation selon l'une quelconque des revendications 1-13, dans laquelle des vecteurs adénoviraux multiples, chaque vecteur adénoviral comprenant une ou plusieurs séquences d'acide nucléique codant pour un ou plusieurs antigènes, doivent être administrés au mammifère.

15. Utilisation selon l'une quelconque des revendications 1-14, dans laquelle le vecteur adénoviral comprend une protéine d'enveloppe adénovirale chimérique comprenant une séquence d'acides aminés non native codant pour un antigène.

16. Utilisation selon la revendication 15, dans laquelle au moins un antigène est présenté via des complexes majeurs d'histocompatibilité de type I (CMH I) et au moins un antigène est présenté via des complexes CMH de type II.

17. Utilisation selon l'une quelconque des revendications 1-16, dans laquelle le vecteur adénoviral comprend un génome adénoviral dépourvu de séquences d'acide nucléique natives codant pour des protéines adénovirales.

18. Utilisation l'une quelconque des revendications 8-17, dans laquelle une séquence espaceur est positionnée dans la région E4 du génome adénoviral.

19. Utilisation selon l'une quelconque des revendications 1-18, dans laquelle le vecteur adénoviral comprend des séquences d'acide nucléique multiples codant pour différents antigènes.

20. Utilisation selon la revendication 19, dans laquelle deux séquences d'acide nucléique ou davantage codant pour différents antigènes sont en liaison fonctionnelle avec différents promoteurs.

21. Utilisation selon l'une quelconque des revendications 1-18, dans laquelle le vecteur adénoviral comprend des séquences d'acide nucléique multiples codant pour le même antigène.

22. Utilisation selon la revendication 21, dans laquelle deux séquences d'acide nucléique ou davantage codant pour le même antigène sont en liaison fonctionnelle avec différents promoteurs.

23. Utilisation selon l'une quelconque des revendications 1-22, dans laquelle le vecteur adénoviral comprend une protéine d'enveloppe adénovirale chimérique comprenant une séquence d'acides aminés non native, et dans laquelle la séquence d'acides aminés non native comprend un motif RGD.


### SEQUENCE LISTING

<110> GALL, Jason G. D.
   WICKHAM, Thomas J.
   ENRIGHT, William J.
   BROUGH, Douglas E.
   ZUBER, Mohammed
   KING, C. Richter
<120> ADENOVIRAL VECTOR-BASED VACCINES
<130> 229796
<150> US 60/490,106
   <151> 2003-07-25
<150> US 60/588,000
   <151> 2004-07-14
<160> 11
<170> PatentIn version 3.2
<210> 1
   <211> 5
   <212> PRT
   <213> Artificial
<220>
   <223> Synthetic
<400> 1
   Cys Arg Gly Asp Cys
   1               5
<210> 2
   <211> 9
   <212> PRT
   <213> Artificial
<220>
   <223> Synthetic
<220>
   <221> MISC_FEATURE
   <222> (2)..(2)
   <223> "Xaa" may be any amino acid
<220>
   <221> MISC_FEATURE
   <222> (8)..(8)
   <223> "Xaa" may be any amino acid
<400> 2
   Cys Xaa Cys Arg Gly Asp Cys Xaa Cys
   1 5
<210> 3
   <211> 9
   <212> PRT
   <213> Artificial
<220>
   <223> Synthetic
<400> 3
   Cys Asp Cys Arg Gly Asp Cys Phe Cys
   1 5
<210> 4
   <211> 144
   <212> PRT
   <213> Adenovirus
<400> 4
   Met Asn Gly Thr Thr Gln Asn Asn Ala Ala Leu Phe Asp Gly Gly Val
   1 5 10 15
   Phe Ser Pro Tyr Leu Thr Ser Arg Leu Pro Tyr Trp Ala Gly Val Arg
   20 25 30
   Gln Asn Val Val Gly Ser Thr Val Asp Gly Arg Pro Val Ala Pro Ala
   35 40 45
   Asn Ser Ser Thr Leu Thr Tyr Ala Thr Ile Gly Pro Ser Pro Leu Asp
   50 55 60
   Thr Ala Ala Ala Ala Ala Ala Ser Ala Ala Ala Ser Thr Ala Arg Ser
   65 70 75 80
   Met Ala Ala Asp Phe Ser Phe Tyr Asn His Leu Ala Ser Asn Ala Val
   85 90 95
   Thr Arg Thr Ala Val Arg Glu Asp Ile Leu Thr Val Met Leu Ala Lys
   100 105 110
   Leu Glu Thr Leu Thr Ala Gln Leu Glu Glu Leu Ser Gln Lys Val Glu
   115 120 125
   Glu Leu Ala Asp Ala Thr Thr His Thr Pro Ala Gln Pro Val Thr Gln
   130 135 140
<210> 5
   <211> 125
   <212> PRT
   <213> Adenovirus
<400> 5
   Met Ala Glu Glu Gly Arg Ile Tyr Val Pro Tyr Val Thr Ala Arg Leu
   1               5                   10                  15
   Pro Lys Trp Ser Gly Ser Val Gln Asp Lys Thr Gly Ser Asn Met Leu
               20                  25                  30
   Gly Gly Val Val Leu Pro Pro Asn Ser Gln Ala His Arg Thr Glu Thr
           35                  40                  45
   Val Gly Thr Glu Ala Thr Arg Asp Asn Leu His Ala Glu Gly Ala Arg
       50                  55                  60
   Arg Pro Glu Asp Gln Thr Pro Tyr Met Ile Leu Val Glu Asp Ser Leu
   65                  70                  75                  80
   Gly Gly Leu Lys Arg Arg Met Asp Leu Leu Glu Glu Ser Asn Gln Gln
                   85                  90                  95
   Leu Leu Ala Thr Leu Asn Arg Leu Arg Thr Gly Leu Ala Ala Tyr Val
               100                 105                 110
   Gln Ala Asn Leu Val Gly Gly Gln Val Asn Pro Phe Val
           115                 120                 125
<210> 6
   <211> 125
   <212> PRT
   <213> Adenovirus
<400> 6
   Met Ala Glu Glu Gly Arg Ile Tyr Val Pro Tyr Val Thr Ala Arg Leu
    1               5                  10                  15
   Pro Lys Trp Ser Gly Ser Val Gln Asp Lys Thr Gly Ser Asn Met Leu
               20                  25                  30
   Gly Gly Val Val Leu Pro Pro Asn Ser Gln Ala His Arg Thr Glu Thr
           35                  40                  45
   Val Gly Thr Glu Ala Thr Arg Asp Asn Leu His Ala Glu Gly Ala Arg
       50                  55                  60
   Arg Pro Glu Asp Gln Thr Pro Tyr Met Ile Leu Val Glu Asp Ser Leu
   65                  70                  75                  80
   Gly Gly Leu Lys Arg Arg Met Asp Leu Leu Glu Glu Ser Asn Gln Gln
                   85                  90                  95
   Leu Leu Ala Thr Leu Asn Arg Leu Arg Thr Gly Leu Ala Ala Tyr Val
               100                 105                 110
   Gln Ala Asn Leu Val Gly Gly Gln Val Asn Pro Phe Val
           115                 120                 125
<210> 7
   <211> 140
   <212> PRT
   <213> Adenovirus
<400> 7
   Met Ser Ala Asn Ser Phe Asp Gly Ser Ile Val Ser Ser Tyr Leu Thr
   1 5 10 15
   Thr Arg Met Pro Pro Trp Ala Gly Val Arg Gln Asn Val Met Gly Ser
   20 25 30
   Ser Ile Asp Gly Arg Pro Val Leu Pro Ala Asn Ser Thr Thr Leu Thr
   35 40 45
   Tyr Glu Thr Val Ser Gly Thr Pro Leu Glu Thr Ala Ala Ser Ala Ala
   50 55 60
   Ala Ser Ala Ala Ala Ala Thr Ala Arg Gly Ile Val Thr Asp Phe Ala
   65 70 75 80
   Phe Leu Ser Pro Leu Ala Ser Ser Ala Ala Ser Arg Ser Ser Ala Arg
   85 90 95
   Asp Asp Lys Leu Thr Ala Leu Leu Ala Gln Leu Asp Ser Leu Thr Arg
   100 105 110
   Glu Leu Asn Val Val Ser Gln Gln Leu Leu Asp Leu Arg Gln Gln Val
   115 120 125
   Ser Ala Leu Lys Ala Ser Ser Pro Pro Asn Ala Val
   130 135 140
<210> 8
   <211> 140
   <212> PRT
   <213> Adenovirus
<400> 8
   Met Ser Thr Asn Ser Phe Asp Gly Ser Ile Val Ser Ser Tyr Leu Thr
   1 5 10 15
   Thr Arg Met Pro Pro Trp Ala Gly Val Arg Gln Asn Val Met Gly Ser
   20 25 30
   Ser Ile Asp Gly Arg Pro Val Leu Pro Ala Asn Ser Thr Thr Leu Thr
   35 40 45
   Tyr Glu Thr Val Ser Gly Thr Pro Leu Glu Thr Ala Ala Ser Ala Ala
   50 55 60
   Ala Ser Ala Ala Ala Ala Thr Ala Arg Gly Ile Val Thr Asp Phe Ala
   65 70 75 80
   Phe Leu Ser Pro Leu Ala Ser Ser Ala Ala Ser Arg Ser Ser Ala Arg
   85 90 95
   Asp Asp Lys Leu Thr Ala Leu Leu Ala Gln Leu Asp Ser Leu Thr Arg
   100 105 110
   Glu Leu Asn Val Val Ser Gln Gln Leu Leu Asp Leu Arg Gln Gln Val
   115 120 125
   Ser Ala Leu Lys Ala Ser Ser Pro Pro Asn Ala Val
   130 135 140
<210> 9
   <211> 132
   <212> PRT
   <213> Adenovirus
<400> 9
   Met Ser Gly Phe Thr Glu Gly Asn Ala Val Ser Phe Glu Gly Gly Val
   1 5 10 15
   Phe Ser Pro Tyr Leu Thr Thr Arg Leu Pro Ser Trp Ala Gly Val Arg
   20 25 30
   Gln Asn Val Val Gly Ser Asn Val Asp Gly Arg Pro Val Ala Pro Ala
   35 40 45
   Asn Ser Thr Thr Leu Thr Tyr Ala Thr Ile Gly Ser Ser Val Asp Thr
   50 55 60
   Ala Ala Ala Ala Ala Ala Ser Ala Ala Ala Ser Thr Ala Arg Gly Met
   65 70 75 80
   Ala Ala Asp Phe Gly Leu Tyr Asn Gln Leu Ala Ala Ser Arg Leu Arg
   85 90 95
   Glu Glu Asp Ala Leu Ser Val Val Leu Thr Arg Leu Glu Glu Leu Ser
   100 105 110
   Gln Gln Leu Gln Asp Met Ser Ala Lys Met Ala Leu Leu Asn Pro Pro
   115 120 125
   Ala Asn Thr Ser
   130
<210> 10
   <211> 133
   <212> PRT
   <213> Adenovirus
<400> 10
   Met Ser Gly Ser Met Glu Gly Asn Ala Val Ser Phe Lys Gly Gly Val
    1               5                  10                  15
   Phe Ser Pro Tyr Leu Thr Thr Arg Leu Pro Ala Trp Ala Gly Val Arg
               20                  25                  30
   Gln Asn Val Met Gly Ser Asn Val Asp Gly Arg Pro Val Ala Pro Ala
           35                  40                  45
   Asn Ser Ala Thr Leu Thr Tyr Ala Thr Val Gly Ser Ser Val Asp Thr
       50                  55                  60
   Ala Ala Ala Ala Ala Ala Ser Ala Ala Ala Ser Thr Ala Arg Gly Met
   65                  70                  75                  80
   Ala Ala Asp Phe Gly Leu Tyr Asn Gln Leu Ala Ala Ser Arg Ser Leu
                   85                  90                  95
   Arg Glu Glu Asp Ala Leu Ser Val Val Leu Thr Arg Met Glu Glu Leu
               100                 105                 110
   Ser Gln Gln Leu Gln Asp Leu Phe Ala Lys Val Ala Leu Leu Asn Pro
           115                 120                 125
   Pro Ala Asn Ala Ser
       130
<210> 11
   <211> 166
   <212> DNA
   <213> adeno-associated virus
<400> 11
   ctggaggggt ggagtcgtga cgtgaattac gtcatagggt tagggaggtc ctgtattaga     60
   ggtcacgtga gtgttttgcg acattttgcg acaccatgtg gtcacgctgg gtatttaagc    120
   ccgagtgagc acgcagggtc tccattttga agcgggaggt ttgaac                   166